# EUROPEAN PATENT SPECIFICATION

(11) **EP 2 750 646 B1**
(45) Date of publication and mention of the grant of the patent: **31.01.2018**
(21) Application number: 12827528.6
(22) Date of filing: 23.08.2012
(51) Int. Cl.: A61F 13/49, A61F 13/535, A61F 13/15, A61F 13/45

(54) **METHOD OF MANUFACTURING ABSORBENT ARTICLE**
VERFAHREN ZUR HERSTELLUNG EINES SAUGFÄHIGEN ARTIKELS
PROCÉDÉ DE FABRICATION D'UN ARTICLE ABSORBANT

(30) Priority: 29.08.2011 JP 2011185850
(43) Date of publication of application: 09.07.2014
(73) Proprietor: Unicharm Corporation, Ehime 799-0111 (JP)
(72) Inventor: MUKAI, Hirotomo, Kanonji-shi Kagawa 769-1602 (JP); ARAYAMA, Takaya, Kanonji-shi Kagawa 769-1602 (JP)
(74) Representative: Peter, Julian
(86) International application number: PCT/JP2012/005274
(87) International publication number: WO 2013/031150

(56) References cited:
- EP-A1- 2 260 808
- EP-A1- 2 308 432
- WO-A1-2012/090508
- JP-A- H11 318 977
- JP-A- 2005 323 685
- JP-A- 2006 115 996
- JP-A- 2006 115 996
- JP-A- 2006 149 571
- JP-A- 2006 500 155
- JP-A- 2007 144 104
- JP-A- 2008 023 365
- JP-A- 2010 115 403
- JP-A- 2010 279 612
- JP-A- 2011 136 094
- JP-A- 2012 143 539
- JP-B2- 4 092 319

## Description

### Technical Field

The present disclosure relates to a method of manufacturing an absorbent article including a curving unit which is capable of curving an absorber.

### Background Art

In an absorbent article such as a pants-type diaper, in order to improve the comfort of the wearer when wearing the absorbent article and to prevent leakage of excretions, various methods have been devised. For example, there is known an absorbent article in which a curving unit that acts as a base point for curving the absorber to a wearer's side is formed at the absorber. For example, Japanese Patent Publication No. 4092319, Japanese Unexamined Patent Application Publication No. 2010-273842 and Japanese Patent Publication No. 4587909 describe an absorbent article in which a groove unit and a non fiber-deposited unit is formed along a longitudinal direction of an absorber that absorbs the excretions of a wearer.

The absorber of the absorbent article according to JP 4092319 is configured by superimposing two separate absorption layers. A pair of groove units is formed near the outer ends of the upper absorption layer in the widthwise direction, and a groove unit is formed in the central portion of the lower absorption layer in the widthwise direction. The absorber is configured to fold along the three groove units, and also to curve towards the wearer.

Furthermore, JP 2010-273842 describes a method of manufacturing an absorbent article in which a groove unit is formed in an absorber configured by a single layer. According to the method of manufacturing the absorbent article, in the recessed portion of a forming die in which a recess having the reverse shape of the shape of an absorber is formed, an absorbent material constituting the absorber is deposited to manufacture the absorber. A part of the base surface of the forming die is protruding out, and a non-suction region in which the absorbent material is not sucked in is provided. The portion corresponding to the non-suction region configures the groove unit in which the absorbent material is not deposited.

Furthermore, JP 4587909 describes a method of manufacturing an absorbent article in which a non fiber-deposited unit corresponding to a groove unit is formed in an absorber configured by a single layer. According to the method of manufacturing the absorbent article, an absorbent material constituting the absorber is deposited in a number of concave units for accumulation that are formed on the outer circumference of a rotating drum to configure the absorber. Convex units are formed in the base surface of the concave units for accumulation, and the portion corresponding to the convex units configures a non fiber-deposited unit in which the absorbent material is not deposited.
Further, WO2012/090508 A1 discloses a method for manufacturing an absorbent article including an absorber relevant to the technical field of the invention.

### Citation List

### Patent Literature

PTL1 : Japanese Patent Publication No. 4092319 (Fig. 7 and others)
PTL2: Japanese Unexamined Patent Application Publication No. 2010-273842 (Fig. 16 and others)
PTL 3: Japanese Patent Publication No. 4587909 (Fig. 3, Fig. 4, and others)

### Summary of Invention

However, the inventor(s) has/have recognized that according to the method of manufacturing an absorbent article described in JP 2010-273842 and JP 4587909, a groove unit and a non fiber-deposited unit that act as the base point for deformation of the absorber can be provided in the absorber configured by a single layer. However, if an absorber configured by a single layer gets deformed with a groove unit, for example, as the base point, the basis weight of the absorber in the groove unit, for example, that acts as the base point of deformation is lower as compared to the portion in which a groove unit, for example, is not formed, which might result in a decline in the absorption performance. Therefore, similar to the absorber described in JP 4092319, the formation of a groove unit in each absorption layer of an absorber configured by a plurality of absorption layers, and the deformation of the absorber with the groove units, for example, as a base point is taken in to consideration.

The inventor(s) has/have further recognized that in the disposable absorbent article described in JP 4092319, because a groove unit is formed respectively in the upper absorption layer and the lower absorption layer, and the upper absorption layer and the lower absorption layer are provided independently, the relative position of the grove unit formed in the upper absorption layer and the groove unit formed in the lower absorption layer might get misaligned. If the relative position of groove units formed in each absorption layer gets misaligned, the arrangement of the groove units in the entire absorber deviates, and the interval between the groove units also deviates, and the absorber might not get deformed so as to become symmetrical in the widthwise direction. If the absorber gets deformed asymmetrically, a feeling of discomfort might be felt by the wearer at the time of wearing the absorbent article.

Particularly, because the absorber in the crotch region corresponding to the crotch of the wearer is arranged in a relatively narrow space between the legs of the wearer, when the position of the groove unit deviates and the absorber gets deformed asymmetrically, the feeling of discomfort by the wearer is remarkable. Additionally, because the absorber in the crotch region is adapted to be in contact with a crotch of the wearer, if the crotch region gets deformed asymmetrically, side leakage of the bodily fluid might occur.

A method of manufacturing an absorbent article including an absorber having a longitudinal direction, a widthwise direction perpendicular to the longitudinal direction, an inner direction for facing a wearer, and an outer direction opposite to the inner direction, wherein a curving unit that allows the absorber to curve in the inner direction or the outer direction is formed in the absorber, said method including: a first absorption layer-forming step of forming a first absorption layer configuring the absorber; and a second absorption layer-forming step of forming a second absorption layer configuring the absorber, and laminated on to the first absorption layer, wherein in the first absorption layer-forming step, the first absorption layer is formed with the curving unit in symmetry with respect to a virtual central line dividing a width of the absorber in the widthwise direction into two, and extending in the longitudinal direction, and in the second absorption layer-forming step, the second absorption layer is formed without providing the curving unit in a crotch region that is adapted to be in contact with a crotch of the wearer, wherein the curving unit is configured by a slit formed in the first absorption layer, and wherein in the first absorption layer-forming step, a first curving unit including a first slit extending in the longitudinal direction is formed in the center of the crotch region in the widthwise direction, and a second curving unit including a pair of second slits extending in the longitudinal direction is formed outboard of the first curving unit in the widthwise direction, and a third curving unit including a pair of third slits extending in the longitudinal direction is formed outboard of the second curving unit in the widthwise direction.

### Brief Description of Drawings

[fig. 1] Fig. 1 is a schematic perspective view of a disposable diaper 1 according to at least one embodiment.
[fig. 2] Fig. 2 is an exploded plan view of the disposable diaper 1.
[fig. 3] Fig. 3 is a widthwise-direction cross-sectional view of the disposable diaper 1 along the X1-X'1 line shown in Fig. 2.
[fig. 4] Fig. 4 is a longitudinal-direction cross-sectional view of the disposable diaper 1 along the X2-X'2 line shown in Fig. 2.
[fig. 5] Fig. 5 is a widthwise-direction cross-sectional view of the disposable diaper 1 along the X3-X'3 line shown in Fig. 2.
[fig. 6] Fig. 6 is a plan view of the absorber according to at least one embodiment.
[fig. 7] Fig. 7 is a perspective view of the absorber.
[fig. 8] Fig. 8 is a cross-sectional view along the X1-X'1 line that schematically illustrates the worn state of the disposable diaper 1 according to at least one embodiment.
[fig. 9] Fig. 9 is a cross-sectional view (when the wearer's legs are closed) that schematically illustrates the worn state of the disposable diaper 1.
[fig. 10] Fig. 10 is a diagram for explaining a manufacturing step in a method of manufacturing an absorber in accordance with a first embodiment.
[fig.11]Fig. 11 is a partial perspective view of a manufacturing step including a device for manufacturing an absorber according to the first embodiment.
[fig.12]Fig. 12 is a side view of a manufacturing device illustrating the inside of a device for manufacturing an absorber according to the first embodiment.
[fig.13]Fig. 13 is a partial perspective view of a manufacturing step including a device for manufacturing an absorber according to a second embodiment.
[fig.14]Fig. 14 is a side view of a manufacturing device illustrating the inside of a device for manufacturing an absorber according to the second embodiment.
[fig.15]Fig. 15 plan views of absorbers according to various modifications.
[fig.16]Fig. 16 is cross-section views of the absorbers according to the modifications.

### Description of Embodiments

Next, embodiments of a disposable diaper 1, as an absorbent article according to the present disclosure, is explained with reference to drawings. In the following description of the drawings, the same or similar reference numerals are used to designate the same or similar parts. It will be appreciated that the drawings are schematic representations and are not drawn to scale unless otherwise specified. Moreover, the drawings do not necessarily reflect the actual dimensional relationships and ratios of components. Accordingly, specific dimensions should be determined in consideration of the explanation below. Moreover, relations or ratios among such dimensions may be different from one drawing to another.

A characteristic of the absorber of the disposable diaper according to at least one embodiment is the formation of a first curving unit, a second curving unit, and a third curving unit to enable curving of the absorber in a convex shape.

### (1) Overall Schematic Structure of Disposable Diaper

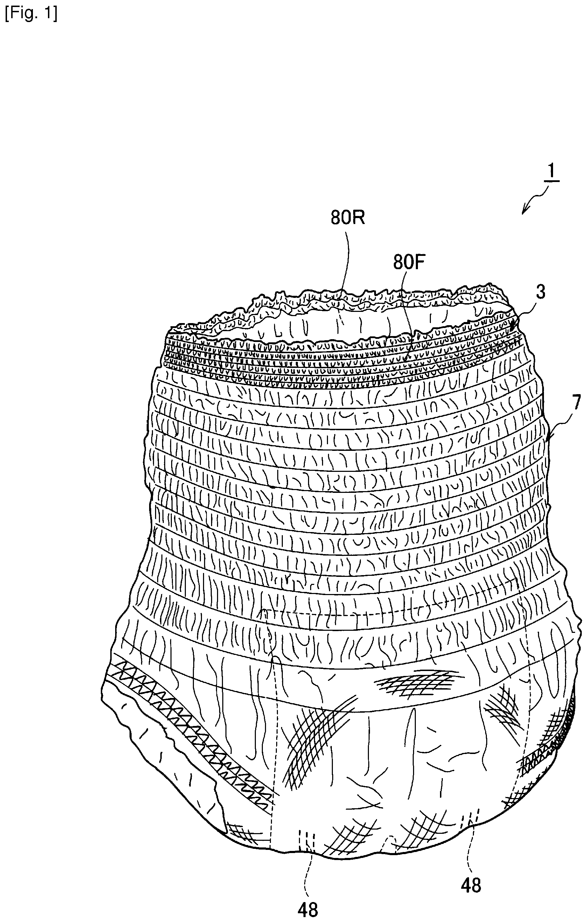

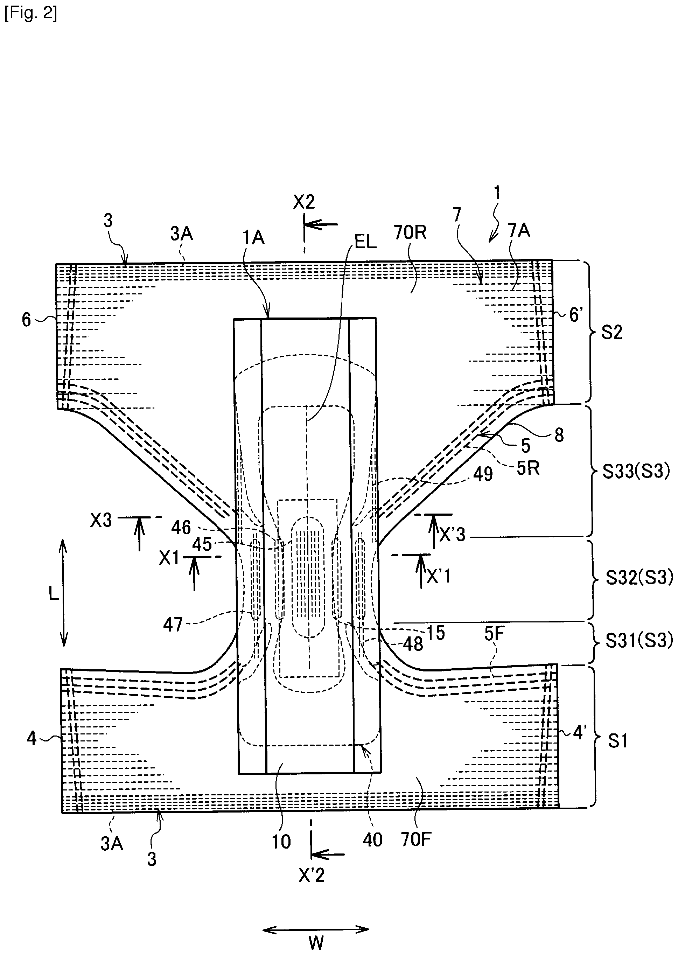

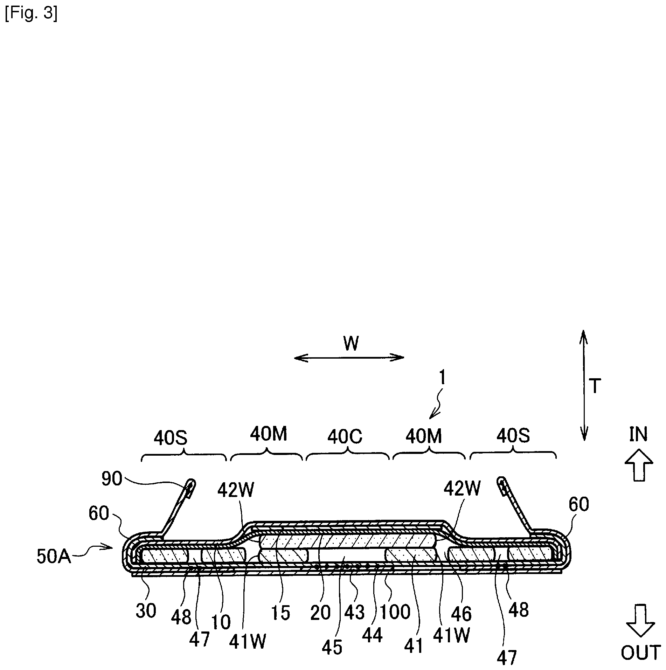

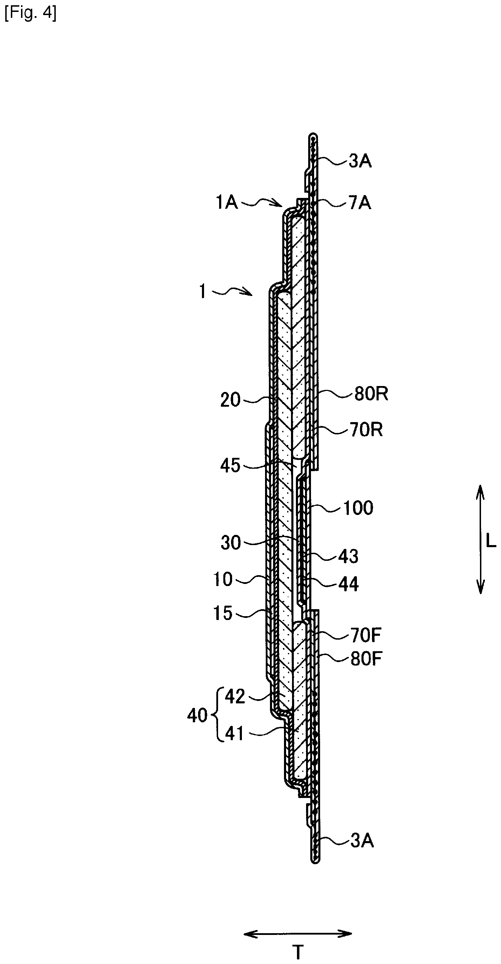

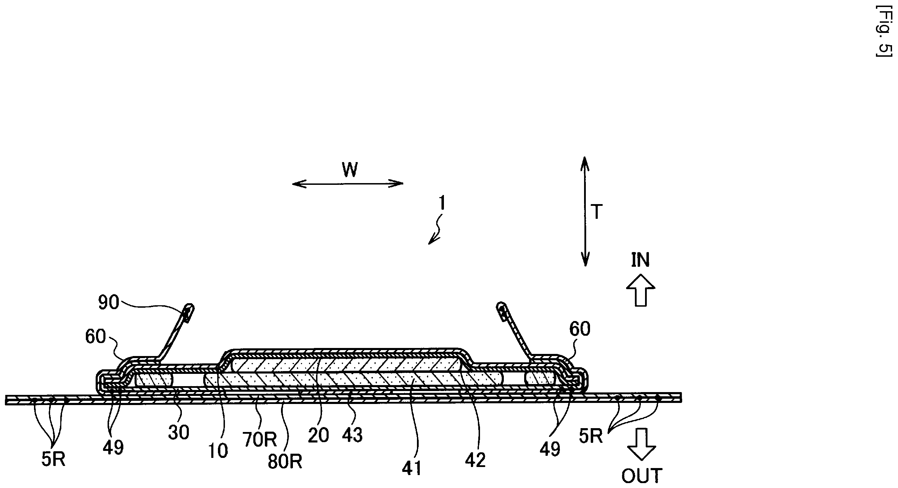

Fig. 1 is a schematic perspective view of the disposable diaper 1 configuring the disposable diaper of at least one embodiment. Fig. 2 is an exploded plan view of the disposable diaper 1. Fig. 3 is a widthwise cross-sectional view of the disposable diaper 1 along the X1 - X'1 line shown in Fig. 2. Fig. 4 is a longitudinal-direction cross-sectional view of the disposable diaper 1 along the X2-X'2 line shown in Fig. 2. Fig. 5 is a widthwise cross-sectional view of the disposable diaper 1 along the X3- X'3 line shown in Fig. 2. The disposable diaper 1 is, for example, a pants-type disposable diaper.

As shown in Fig. 2, the disposable diaper 1 has a front waistline region S1 corresponding to the front waistline of the wearer, a back waistline region S2 corresponding to the back waistline of the wearer, and a crotch region S3 corresponding to the crotch of the wearer and positioned between the front waistline region S 1 and the back waistline region S2, in a longitudinal direction L of the absorbent article 1. The crotch region S3 has a narrow region S32 in which the width in the crotch region of the wearer is the narrowest when the wearer's legs are closed, a middle inside leg portion (ventral side) S31 positioned between the narrow region S32 and the front waistline region S1, and a middle inside leg portion (buttocks side) S33 positioned between the narrow region S32 and the back waistline region S2.

Front waistline edge 4, 4' of the front waistline region S1 are joined with back waistline edges 6, 6' of the back waistline region S2, whereby the disposable diaper 1 is formed in the shape of pants.

The disposable diaper 1 has a topsheet 10, an absorber 40, a sidesheet 60, a foreside exterior topsheet 70F, a backside exterior topsheet 70R, an exterior center sheet 100, a foreside exterior backsheet 80F, and a backside exterior backsheet 80R, and these sheets are joined with each other by an adhesive or by thermal fusion bonding.

The foreside exterior topsheet 70F, the backside exterior topsheet 70R, the foreside exterior backsheet 80F, the backside exterior backsheet 80R, and the exterior center sheet 100 form an exterior sheet configuring the exterior portion of the disposable diaper 1. An absorber 40 configured by cotton-like pulp and highly polymerized absorbent polymer is provided on the inner side (skin contact surface side) of the foreside exterior topsheet 70F, the backside exterior topsheet 70R, and the exterior center sheet 100.

The topsheet 10 is a sheet that forms the skin contact surface that can be in direct contact with the skin of the wearer. The topsheet 10 is formed by a liquid-permeable sheet, such as a hydrophilic nonwoven cloth and fabric, an aperture plastic film, or an aperture hydrophobic nonwoven cloth. The topsheet 10 according to at least one embodiment is configured by a hydrophilic spun bond nonwoven cloth with a basis weight of 20 g/m², and made from polypropylene.

An auxiliary sheet 15 is joined with the non-skin contact surface side of the topsheet 10. An auxiliary sheet 15 is arranged between the topsheet 10 and an absorber topside covering sheet 20. By providing the auxiliary sheet 15, the absorption speed of the bodily fluid can be made faster, and the reverse flow of the bodily fluid after absorption can be prevented.

Because the absorber of the absorbent article according to at least one embodiment has a structure such that the absorber is adhered on to the crotch of the wearer, by preventing the reverse flow of the bodily fluid after absorption, the comfort after excretion can be improved. An air-through nonwoven cloth and a porous film can be used as the auxiliary sheet 15, for example. The auxiliary sheet 15 according to at least one embodiment is formed from a 50 g/ m² (hydrophilic) air-through nonwoven cloth.

In at least one embodiment, the width of a second slit 46 is 10 mm, the interval between the inner ends in the widthwise direction of a pair of the second slits 46 described later is 76 mm, and the width of the auxiliary sheet is 80 mm.

The absorber topside covering sheet 20 is provided between the topsheet 10 and the absorber 40. The absorber topside covering sheet 20 is formed by a liquid-permeable sheet, such as a hydrophilic nonwoven cloth and fabric, an aperture plastic film, an aperture hydrophobic nonwoven cloth, or a tissue. An absorber backside covering sheet 30 is provided in the non-skin contact surface side of the absorber 40. The absorber backside covering sheet 30 is formed by a sheet such as a liquid-impermeable film (for example, polyethylene).

The absorber 40 is provided between the absorber topside covering sheet 20 and the absorber backside covering sheet 30. The absorber 40 has a lengthwise direction L from the front waistline region S1 towards the back waistline region S2, and a widthwise direction W perpendicular to the lengthwise direction L. Additionally, the absorber 40 has an inner direction IN towards the wearer wearing the disposable diaper 1 and an outer direction OUT towards the opposite side of the inner direction. The absorber 40 is formed from a powder mix of ground pulp and/or a highly polymerized absorbent polymer.

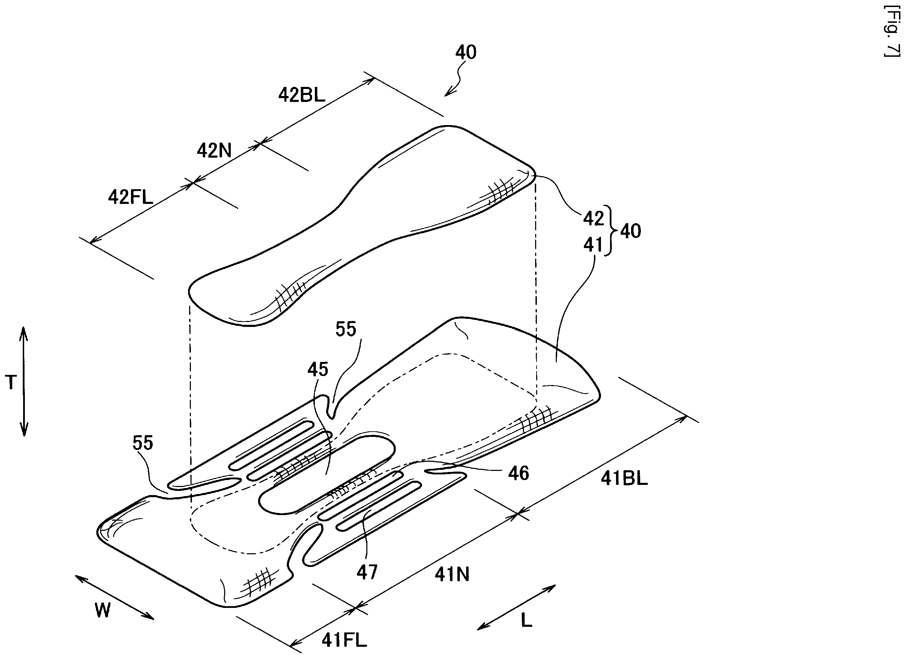

The absorber 40 includes a first absorption layer 41 in which a curving unit is formed in the crotch region S3, and a second absorption layer 42 superimposed with the first absorption layer 41 and in which a curving unit is not formed in the crotch region S3. According to the absorber 40 of at least one embodiment, the first absorption layer 41 is arranged in the non-skin contact surface side of the absorber, and the second absorption layer 42 is arranged in skin contact surface side of the absorber (see Fig. 7).

A first slit 45 configuring the first curving unit is formed in the center of the first absorption layer 41 in the widthwise direction W. The pair of second slits 46 configuring the second curving unit is positioned outboard of the first slit 45 in the widthwise direction. A pair of third slits 47 configuring the third curving unit is positioned outboard of the pair of second slits 46 in the widthwise direction.

The absorbent article 1 has a first elastic member 44 arranged in a thickness direction T of the absorbent article 1 so as to overlap the first slit 45, and a second elastic member 48 arranged in the thickness direction T such that at least a part of the second elastic member 48 overlaps the third slits 47.

As a result of the elastic materials and slits formed in the absorber 40, the absorber 40 is configured to curve when the disposable diaper 1 is worn. In at least one embodiment, the first elastic member 44 and the first slit 45 configure the first curving unit, the second slits 46 configure the second curving unit, and the third slits 47 and the second elastic member 48 configure the third curving unit.

The curving unit in the absorber of at least one embodiment is the slit formed in the first absorption layer 41. Additionally, the curving unit according to at least one embodiment is configured by the slit formed in the absorber and an elastic member, however, rather than providing an elastic member, the curving unit may be configured only by the slit formed in the absorber.

The curving unit, such as the slit, is arranged symmetrically with respect to a virtual central line EL dividing the width of the absorber in the widthwise direction into two and extending in the longitudinal direction, in the crotch region S3. A configuration of the absorber 40 is explained later in detail.

The sidesheets 60 are provided on both ends of the absorber 40 in the widthwise direction W so as to wrap the topsheet 10, the absorber topside covering sheet 20, and the absorber backside covering sheet 30 as one part. The sidesheets 60 are formed by sheets of liquid-impermeable nonwoven cloth, and a leakage-preventing wall for preventing the side leakage of excretions is configured by the sidesheets 60 and the side elastic material 90.

The exterior topsheet includes the foreside exterior topsheet 70F formed in the front waistline region S1 and the middle inside leg portion (ventral side) S31, and the backside exterior topsheet 70R formed in the back waistline region S2 and the middle inside leg portion (buttocks side) S33. In the thickness direction T, the foreside exterior topsheet 70F is arranged between the foreside exterior backsheet 80F and the absorber 40. In the thickness direction T, the backside exterior topsheet 70R is arranged between the backside exterior backsheet 80R and the absorber 40. In the longitudinal direction L, the exterior center sheet 100 is arranged between the foreside exterior topsheet 70F and the backside exterior topsheet 70R.

The front end of the exterior center sheet 100 is joined with the back end of the foreside exterior topsheet 70F, and the back end of the exterior center sheet 100 is joined with the front end of the backside exterior topsheet 70R. The exterior center sheet 100 is arranged across the foreside exterior topsheet 70F and the backside exterior topsheet 70R. The exterior center sheet 100 is joined with the top side of the exterior topsheet by a hot-melt adhesive coated continuously by a slot coater.

The exterior center sheet 100 is configured by a nonwoven fabric, for example. The exterior center sheet according to at least one embodiment is configured by an SMS (spunbond-meltblown-spunbond) nonwoven cloth with a basis weight of 15 g/ m², and made from polypropylene. When wearing the disposable diaper 1g, the exterior center sheet 100 is positioned towards the inner side (skin contact surface side) from the exterior topsheet.

The foreside exterior topsheet 70F and the backside exterior topsheet 70R are formed in the front waistline region S1 and the back waistline region S2 such that the width in the widthwise direction W is more than any other region. The foreside exterior topsheet 70F and the backside exterior topsheet 70R can be formed by an air-through nonwoven cloth, a spun bond nonwoven cloth, an SMS nonwoven cloth, or a water-resistive film. The exterior topsheet according to at least one embodiment is configured by an SMS nonwoven cloth with a basis weight of 15 g/ m² and made from polypropylene.

The foreside exterior backsheet 80F is provided towards the non-skin contact surface side from the foreside exterior topsheet 70F, in the front waistline region S1. The backside exterior backsheet 80R is provided towards the non-skin contact surface side from the backside exterior topsheet 70R, in the back waistline region S2. One end of the foreside exterior backsheet 80F (backside exterior backsheet 80R) in the longitudinal direction L is folded back towards the skin contact surface side, and is provided so as to wrap the ends of the foreside exterior topsheet 70F (backside exterior topsheet 70R) in the longitudinal direction L.

The foreside exterior backsheet 80F and the backside exterior backsheet 80R can be formed by an air-through nonwoven cloth, a spun bond nonwoven cloth, an SMS nonwoven cloth, or a water-resistive film. The foreside exterior backsheet 80F and the backside exterior backsheet 80R according to at least one embodiment are configured by a spun bond nonwoven cloth with a basis weight of 17 g/ m², and made from polypropylene.

The exterior center sheet 100, the foreside exterior topsheet 70F, the backside exterior topsheet 70R, the foreside exterior backsheet 80F, and the backside exterior backsheet 80R configuring the exterior sheet may be liquid permeable or liquid impermeable.

The absorber backside covering sheet 30 is bonded partially with the foreside exterior topsheet 70F, the backside exterior topsheet 70R, and the exterior center sheet 100.

Waist gathers 3 and waistline gathers 7 are provided in the front waistline region S1 and the back waistline region S2. The waist gathers 3 and the waistline gathers 7 have elongated waist elastic members 3A and waistline elastic members 7A of synthetic rubber that are laid out to stretch and contract along the widthwise direction W of the absorber 40. The waist elastic members 3A and the waistline elastic members 7A are joined between the foreside exterior topsheet 70F and the foreside exterior backsheet 80F, and also between the backside exterior topsheet 70R and the backside exterior backsheet 80R with an adhesive (for example, hot melt adhesive) in a stretched state along the widthwise direction W of the disposable diaper 1.

The waist gathers 3 and the waistline gathers 7 continue from one front waistline edge 4 up to the other front waistline edge 4' in the front waistline region S1, and from one back waistline edge 6 up to the other front waistline edge 6' in the back waistline region S2.

Leg gathers are formed in the middle inside leg edge 8 of the backside exterior backsheet 80R. The leg gathers are formed to run along the leg portions of the wearer. The leg gathers are formed from elongated leg hole elastic members 5 made of synthetic rubber and laid out to stretch and contract. The leg hole elastic members 5 are configured by the front leg hole elastic members 5F arranged in the middle inside leg portion (ventral side) S31 from the front waistline region S1, and the back leg hole elastic members 5R arranged in the middle inside leg portion (buttocks side) S33 from the back waistline region S2.

Both the sidesheets overlap at the end of the sidesheets 60 in the widthwise direction. The side elastic members 90 (see Fig. 3) are provided in a stretched state along the longitudinal direction L, in the portion where the sidesheets overlap each other. The side elastic members 90 continue from the middle inside leg portion (buttocks side) S33 up to the middle inside leg portion (ventral side) S31 via the narrow region S32. The side elastic material 90 is formed by synthetic rubber, etc. having elasticity.

The first elastic member 44 is provided along the longitudinal direction L, and is provided at a position overlapping the first slit 45 in the thickness direction T of the disposable diaper 1. The first elastic member 44 is formed in a convex shape in the inner direction IN, that is, the first elastic member 44 is formed so as to overlap the absorber 40 along the longitudinal direction L such that the absorber 40 curves in a convex shape towards the wearer. The first elastic member 44 is arranged in a stretched state along the longitudinal direction in the center of the absorbent article in the widthwise direction. The first elastic member 44 is arranged from the middle inside leg portion (ventral side) S31 towards the middle inside leg portion (buttocks side) S33, with the narrow region S32 as the center.

The second elastic member 48 is provided at a position overlapping the third slits 47 in the thickness direction T of the disposable diaper 1, in the longitudinal direction L. Two second elastic members 48 are arranged in parallel in the widthwise direction W. The second elastic members 48 are formed in a convex shape in the inner direction IN, that is, the second elastic members 48 are formed so as to overlap the absorber 40 along the longitudinal direction L such that the absorber 40 curves in a convex shape towards the wearer.

The first elastic member 44 is provided between the elastic member covering sheet 43 and the absorber backside covering sheet 30, in a stretched state. The first elastic member 44 is arranged at a percentage of stretch of 1.2 to 2.5 times. Seven first elastic members according to at least one embodiment are stretched and fixed at a percentage of stretch of 1.8 times, and a thickness of 620 dtex. The interval of the first elastic members is 5 mm. The length of the first elastic members 44 in the longitudinal direction L is approx. 120 mm.

The material of the first elastic member 44 is spandex, and the hot-melt adhesive is coated by the V-slot method. An elastic nonwoven fabric, for example, may be used as the first elastic member 44. The elastic member covering sheet 43 is configured by a sheet of a nonwoven fabric, for example, and in at least one embodiment, an SMS nonwoven cloth (hydrophilic) with a basis weight of 15 g/ m², and made from polypropylene is used.

The second elastic member 48 is joined between the absorber backside covering sheet 30 and the sidesheet 60, by an adhesive. Two second elastic members 48 according to at least one embodiment are stretched and fixed at a percentage of stretch of 2.0 times, and a thickness of 620 dtex. The material of the second elastic members 48 is spandex. The hot-melt adhesive is coated on the second elastic members 48 by a direct coating method through slit nozzle coating.

The second elastic members 48 are configured so as to have a stretching stress lesser than that of the first elastic members 44 in a state prior to when the absorber 40 is curved in a convex shape. In other words, the state prior to when the absorber curves in a convex shape is the state in which the worn article is extended in a plane, as shown in Fig. 2. By configuring the second elastic members 48 such that the stretching stress is lesser than that of the first elastic members 44, the height of the convex portion formed by the first elastic members 44 becomes more than that of the convex portion formed by the second elastic members 48. Therefore, the convex portion of the central region can be brought into close contact with the excretion portion of the wearer, when wearing the disposable diaper 1.

Examples of the material of the first elastic members 44 and the second elastic members 48 include, for example, synthetic rubber such as styrene-butadiene, butadiene, isoprene, and neoprene, natural rubber, EVA, elastic polyolefin, spandex, and foamed polyurethane. Other materials of the first elastic members 44 and the second elastic members 48 include an elastic sheet such as an elastic nonwoven fabric.

A side end elastic members 49 are joined between the absorber backside covering sheet 30 and the sidesheet 60 by an adhesive, on both sides of the absorber in the widthwise direction. The side end elastic members 49 are arranged along the longitudinal direction L, across the middle inside leg portion (buttocks side) S33 and the narrow region S32 of the crotch region S3. The side end elastic members 49 are arranged posterior to the second elastic members 48 in the longitudinal direction and outboard of the second elastic members 48 in the widthwise direction. The side end elastic members 49 pull up the sides of the absorber posterior to the narrow region towards the wearer, and deform the absorber so as to encompass the buttocks of the wearer. By thus deforming the absorber, the leakage from the middle inside leg portion (buttocks) can be prevented. Two side end elastic members 49 according to at least one embodiment are stretched and fixed at a percentage of stretch of 2.3 times, and a thickness of 780 dtex.

The expansion stress of the elastic member can be measured as described below, for example.
(1) The material holding the elastic members in between is cut such that all the elastic members forming the convex-shaped portion(s) are included in the widthwise direction. More specifically, from the wearing article according to at least one embodiment, a test piece of 13-mm width x 100-mm length that hold in between three units each of the first elastic members, the second elastic member, and the side end elastic members arranged in an interval of 5 mm, in an elongated state such that there is no sagging, is cut out. In the elongated state, a mark is put at 10 mm from either end in the longitudinal direction of the test piece. A tensile tester made by Instron Japan Co., Ltd. (for example, model No. 5564) or an autograph made by Shimadzu Corporation (for example, model No. AGS-1kNG) can be used to measure the elongation stress.
(2) The test piece prepared at (1) is held in between the upper chuck, such that one of the marks is at the inner end of the upper chuck, and the lower chuck, such that the other mark is at the inner end of the lower chuck. The length of the test piece between the chucks is 80 mm. Note that if the effective length of the gathers of the elastic member is less than 100 mm, a length that is 20 mm shorter than the shortest length from among the effective lengths of the gathers of the elastic members is set as the length of the test piece between the chucks. The initial distance between chucks is set shorter than the length (natural length) when the test piece is relaxed such that no external tension is exerted on the test piece. In order to be alienated from each other, the chucks pull the test piece in the vertical direction under a condition of 100 mm/ min, and elongate the test piece.
(3) By assuming the length of the test piece between the chucks as 100% when the material holding the elastic members in between is elongated without any sagging, the test piece is elongated from the initial distance until its length between the chucks becomes 90%, and then the elongation stress of the test piece is measured for that point of time and set as the stress of the elastic member. That is, in the above embodiment, the elongation stress is measured when the test piece is elongated until its length becomes 90% that is 72 mm, of the 100% length of 80 mm.

The thickness of the absorber 40 is measured by holding the portion to be measured in the thickness measuring gauge in a state when it has been extended to the product length and product width of the absorber 40 (that is, in a flat state such that no creases are formed). A thickness gauge manufactured by PEACOCK (measuring portion: 5-mm diameter, pressure during measurement: 163 g/cm²), for example, can be used as the usable measurement device.

Note that each member configuring the disposable diaper 1 may, for example, use the respective materials described in the Japanese Published Unexamined Application No. 2006-346439, which is incorporated by reference herein in its entirety.

### (2) Structure of the absorber

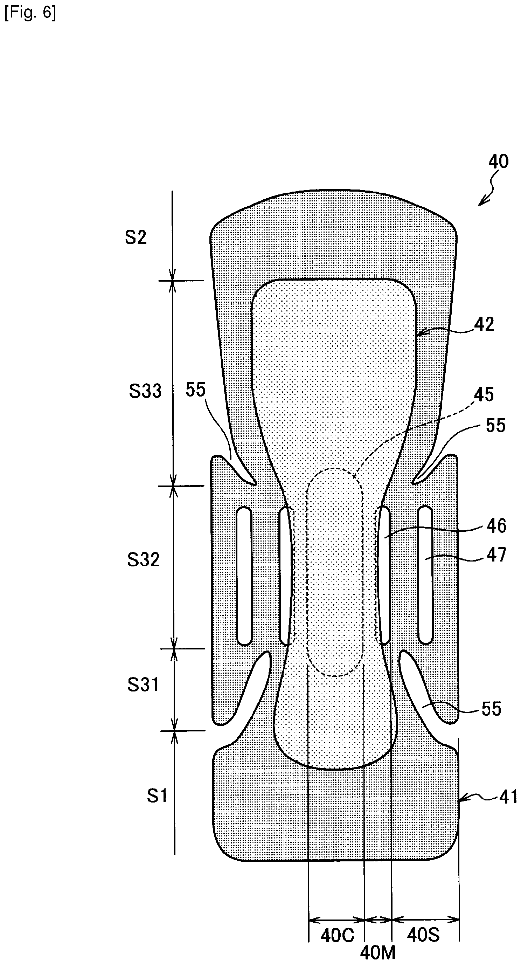

Fig. 6 is a plan view of the absorber 40 and Fig. 7 is a perspective view of the absorber 40. As shown in Fig. 6 and Fig. 7, the absorber 40 includes the first absorption layer 41 in which a curving unit is formed, and the second absorption layer 42 superimposed with the first absorption layer 41, and in which a curving unit is not formed. The first absorption layer 41 is positioned at the non-skin contact surface side of the wearer, and the second absorption layer 42 is positioned at the skin contact surface side of the wearer. The length of the first absorption layer 41 in the longitudinal direction is more than the length of the second absorption layer 42 in the longitudinal direction. The first absorption layer 41 is arranged from the front waistline region S1 across the back waistline region S2.

The first absorption layer 41 and the second absorption layer 42 are configured by cotton-like pulp fibers and highly polymerized absorbent polymer (SAP). The first absorption layer 41 and the second absorption layer 42 can be formed by mixing 0 to 500 g/ m² of pulp and 0 to 500 g/ m²of SAP, for example.

The first absorption layer 41 according to at least one embodiment is formed by mixing 250 g/ m² of pulp and 150 g/ m² of SAP, and has a thickness of approx. 2.5 mm. The second absorption layer 42 according to at least one embodiment is formed by mixing 200 g/ m² of pulp and 90 g/ m² of SAP, and has a thickness of approx. 2.0 mm.

The second absorption layer 42 has a narrow portion 42N that is concave towards the center in the widthwise direction W and has a predetermined width in the widthwise direction W, and a wide portion 42FL and a wide portion 42BL formed on both ends of the narrow portion 42N in the longitudinal direction L, and having a larger width than the width of the narrow portion. The narrow portion 42N is formed in the narrow region S32 of the crotch region S3. The wide region 42FL is formed in the middle inside leg portion (ventral side) S31, and the wide unit 42BL is formed in the middle inside leg portion (buttocks side) S33. The side ends of the narrow portion 42N and the side ends of the wide units 42FL and 42BL are connected by a curved line, and the second absorption layer 42 has a planar shape in the form of an hourglass.

The slit configuring the curving unit is not formed in the crotch region S3 of the second absorption layer. However, the second absorption layer suffices to configure such that a curving unit is not formed in the crotch region, a curving unit may be formed in the front waistline region S1 and the back waistline region S2 other than the crotch region, in the second absorption layer.

The width of the first absorption layer 41 in the widthwise direction decreases from the middle inside leg portion (ventral side) S31 towards the narrow region S32, and the width of the first absorption layer 41 in the widthwise direction decreases from the middle inside leg portion (buttocks side) S33 towards the narrow region S32.

Recessed units 55 are formed in the middle inside leg portion (ventral side) S31 and the middle inside leg portion (buttocks side) S33 of the first absorption layer 41, to be concaved inwardly in the widthwise direction.

The first slit 45, the pair of second slits 46, and the pair of third slits 47 are formed in the first absorption layer 41. The first slit 45 is formed in the central portion in the widthwise direction W. The first slit 45 has a longitudinally-elongated shape extending along the longitudinal direction L, and is formed across the narrow region S32, the middle inside leg portion (ventral side) S31, and the middle inside leg portion (buttocks side) S33. By thus forming the first slit 45, the central portion 40C can be easily curved in a convex shape in the inner direction IN toward the wearer. Furthermore, by improving the diffusive property of the bodily fluid in the crosswise direction of the absorber, and by diffusing the bodily fluid in a wide range, the absorption performance can be improved.

The second slits 46 and the third slits 47 are formed outboard of the first slit 45 in the widthwise direction. The second slits 46 and the third slits 47 have a longitudinally elongated shape extending along the longitudinal direction L, and are formed in the narrow region S32.

The pair of second slits 46 is formed in a convex shape in the outer direction OUT, that is, the pair of second slits 46 is formed in the absorber 40 along the longitudinal direction L such that the absorber 40 curves in a convex shape opposite to that of the first slit 45. The pair of third slits 47 are formed in a convex shape in the inner direction IN, that is, the pair of third slits 47 is formed in the absorber 40 along the longitudinal direction L such that the absorber 40 curves in the same convex shape as the first slit 45.

The width of the absorber, in at least one embodiment, is between 120 and 250 mm in the front waistline region S1 and the back waistline region 2, and between 120 mm and 250 mm in the crotch region S3. The width of the absorber according to at least one embodiment is 196 mm in either region.

The length of the first slit 45 in the longitudinal direction is more than the length of the second slits 46 in the longitudinal direction, and is also more than the length of the third slits 47 in the longitudinal direction. The length of the first slit 45 in the widthwise direction is more than the length of the second slits 46 in the widthwise direction, and is also more than the length of the third slits in the widthwise direction. In at least one embodiment, the width of the first slit is 40 mm, and the width of the second slit and the third slit is 10 mm, respectively.

In at least one embodiment, when the distance from the center of the first slit 45 in the widthwise direction up to the inner ends of the second slits 46 in the widthwise direction is assumed as a, the distance from the outer ends of the second slits 46 in the widthwise direction up to the inner ends of the third slits 47 in the widthwise direction is assumed as b, and the distance from the outer ends of the third slits 47 in the widthwise direction up to the outer ends of the absorber in the widthwise direction is assumed as c, then a = 38 mm, b = 20 mm, and c = 20 mm.

That is, the distance between the first elastic members 44 configuring the first curving unit and the second slits 46 configuring the second curving unit, in the widthwise direction, is more than the distance between the second slits 46 and the third slits 47 configuring the third curving unit, in the widthwise direction. Therefore, the height of the convex portion of the first curving unit is more than the height of the convex portion of the third curving unit. As a result, when the disposable diaper 1 is worn, the first curving unit that takes a convex shape towards the excretion portion of the wearer easily comes in contact with the excretion portion. Furthermore, because the second curving unit forms a concave unit, the excretions can easily enter the concave unit, and the skin of the wearer can be prevented from coming in direct contact with the excretions.

In the crotch region S3, the outer ends 42W in the widthwise direction of the second absorption layer 42 are arranged along the longitudinal direction. The portions of the absorber 40 outboard of the outer ends 42W in the widthwise direction are configured only by the first absorption layer 41, and the portions of the absorber 40 inboard of the outer ends 42W in the widthwise direction are configured by the first absorption layer 41 and the second layer 42, excluding the portion in which the first slit 45 is formed. Therefore, the rigidity and thickness of the absorber 40 changes with the outer ends 42W of the second absorption layer 42 as the boundary. According to at least one embodiment, the absorber curves with the outer ends 42W of the second absorption layer, where the rigidity, etc. change, as the boundary.

The outer ends 42W of the second absorption layer 42 overlap the ends 41W of the first absorption layer in which the second slits 46 are formed, in a thickness direction. With the outer ends 42W of the second absorption layer 42 and the ends 41W of the first absorption layer as the base point, the absorber curves in a convex shape towards the outer direction. The outer ends 42W in the widthwise direction of the second absorption layer 42 and the ends 41W of the first absorption layer run along the longitudinal direction. Therefore, the second curving unit is formed along the longitudinal direction.

The absorber 40 thus configured by the first absorption layer 41 and the second absorption layer 42 has a central portion 40C, an intermediate portion 40M, and side end portions 40S, as shown in Fig. 3. The central portion 40C is formed in the center of the absorber 40 in the widthwise direction W. The intermediate portion is located between the central portion 40C and the side end portions 40S. A convex portion is formed in the central portion 40C by the first curving unit. A convex portion is formed in the intermediate portion 40M by the second curving unit. A convex portion is formed in the side end portions by the third curving unit.

According to at least one embodiment, the first absorption layer 41 and the second absorption layer 42 are formed as one by being pressed in the thickness direction T. The first absorption layer 41 and the second absorption layer 42 may also be formed as one by an adhesive and thermal fusion bonding. Furthermore, in the absorber 40, the first absorption layer 41 is positioned in the non-skin contact surface side and the second absorption layer 42 is positioned in the skin contact surface side, but the second absorption layer 42 may be positioned in the non-skin contact surface side and the first absorption layer 41 may be positioned in the skin contact surface side.

### (3) Changes in the shape of the absorber

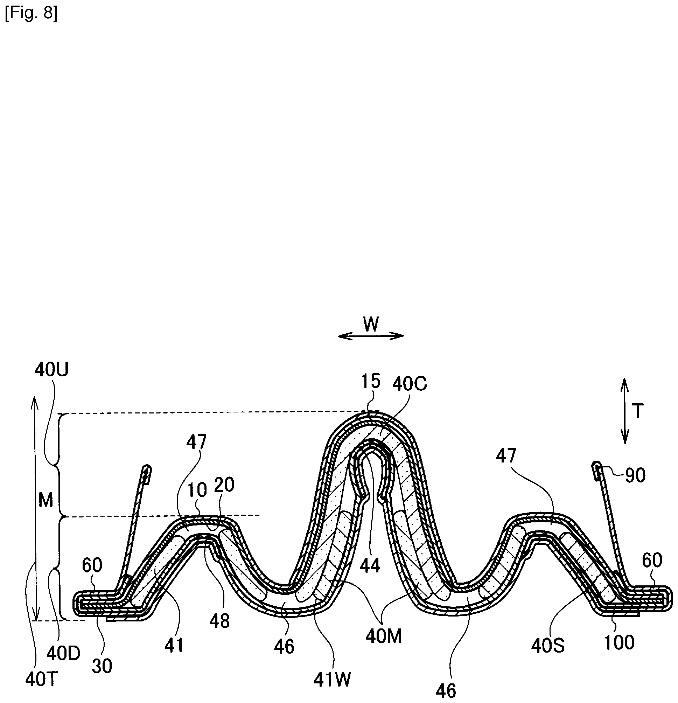

Fig. 8 is a cross-sectional view (with reference to the X1-X'1 line of Fig. 1) that schematically illustrates the worn state of the disposable diaper 1. As shown in Fig. 8, when the disposable diaper 1 is worn, the crotch region S3 of the absorber comes in contact with the crotch of the wearer. In the absorber, a pressure is exerted inwardly in the widthwise direction, by the legs of the wearer. The absorber 40 curves with the first elastic member 44 and the first slit 45, the second slit 46, the third slits 47, and the second elastic member 48 as the base points, and the cross-sectional shape of the disposable diaper 1 along the widthwise direction W gets deformed in a wave shape. Thus, the narrow region S32 of the crotch region S3 of the absorber 40 is in a regular folded state.

As for the absorber 40, the top surface of the absorber 40 that takes a convex shape in the inner direction IN as a result of the first elastic member 44 comes in contact with the crotch of the wearer. Furthermore, the central portion 40C is positioned in the upper region 40U closer to the body of the wearer from the virtual line M dividing the height 40T of the deformed absorber 40 into two. On the other hand, the intermediate portion 40M and the side end portions 40S are positioned in the lower region 40D away from the body of the wearer from the virtual line M.

The central portion 40C in which a convex portion is formed by the first curving unit is configured only from the second absorption layer 42, and is relatively thin. The first absorption layer 41 and the second absorption layer 42 overlap between the convex potion formed by the first curving unit and the convex portion formed by the second curving unit in the intermediate portion 40M, and the intermediate portion has relatively more thickness and higher rigidity. The convex portion formed by the first curving unit can be supported by the portion between the first curving unit and the second curving unit having a high rigidity, and the stability of the convex shape formed by the first curving unit can be improved.

The side edges (leg standing gathers) including the side elastic members 90 are positioned in at least one embodiment at a higher position than the third slits 47 configuring the third curving unit, that is, towards the wearer, in the thickness direction T.

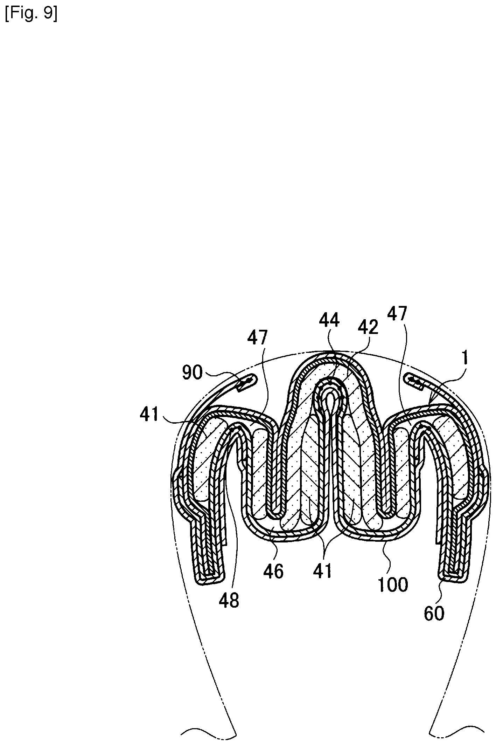

Fig. 9 is a cross-sectional view (with reference to the X1-X'1 line of Fig. 2) that schematically illustrates the worn state of the disposable diaper 1 when the wearer's legs are closed. Note that the virtual line in the figure shows the crotch and both legs of the wearer.

When the wearer closes both legs, the cross-sectional shape of the disposable diaper 1 changes from the state shown in Fig. 8 to the state shown in Fig. 9. When the wearer closes both legs, the absorber folds at the first curving unit, the second curving unit, and the third curving unit, and is arranged compactly at the lower side of the crotch portion with the convex portions formed by the curving units adhering with each other.

At this time, the convex portion formed by the first curving unit formed from the first elastic member 44 and the first slit 45 is positioned to be in contact with the crotch of the wearer. On the other hand, the convex portion formed by the second curving unit formed from the second slits 46 has a convex shape towards the non-skin contact surface side, and is positioned so as not to be in contact with the excretion portion of the wearer. Finally, the convex portion formed by the third curving unit formed from the third slits 47 is lower than the convex portion formed by the first curving unit, and is positioned at the lower side of the crotch of the wearer.

Because the absorber adheres on to the crotch of the wearer, even in cases when the flow of the excreted urine is slow such that it trickles along the skin, the leakage of the bodily fluid can be prevented. Furthermore, in the folded state, a recessed portion extending in the longitudinal direction is formed in the curving unit of the absorber that is away from the skin, and therefore, the bodily fluid can be diffused forwardly or rearwardly in the longitudinal direction, and side leakage can be prevented.

Furthermore, when the disposable diaper 1 is worn, the portion of the absorber 40, corresponding to the side end elastic members 49 is shifted up towards the body of the wearer. The second elastic members 48 are not provided between the side end elastic members in the widthwise direction W. Therefore, the absorber 40 does not get deformed into a convex shape due to the second elastic members 48, for example, but takes a curved shape along the hip portion.

Because of the folding along the first slit 45, the second slits 46, and the third slits 47 formed in the absorber as the base points, as compared to the case when a thin portion is formed in the absorber 40 as a convex portion, the absorber 40 curves easily even when the absorber 40 swells up due to the absorption of liquids. Furthermore, the cross-sectional shape when the absorber 40 gets deformed after the disposable diaper 1 is worn is a tapered shape narrowing towards the skin contact surface side from the non-skin contact surface side. Specifically, the shape of the absorber tapers from the lower region 40D positioned below the virtual line M dividing the height 40T of the deformed absorber 40 into two, towards the upper region 40U closer to the upper side (wearer side) from the virtual line M, and the absorber can be fitted well inside the gap in the crotch of the wearer, without any feeling of discomfort.

Furthermore, the convex portion formed by the first curving unit is configured only from the second absorption layer 42, and is thinner than the portion configured by laminating the first absorption layer 41 and the second absorption layer 42. Due to a thin and high structure, the convex portion formed by the first curving unit can be inserted easily in the thin gap of the crotch portion, and easily adheres on to the excretion portion. Therefore, because the excretion portion and the absorber come in close contact, body fluid can be absorbed rapidly. Furthermore, because the absorber is folded such that the thickness of the absorber is less in the portion close to the skin of the crotch of the wearer, and the thickness is more in the portion away from the skin, the absorber can be fitted without a feeling of discomfort.

### (4) Method of manufacturing the disposable diaper

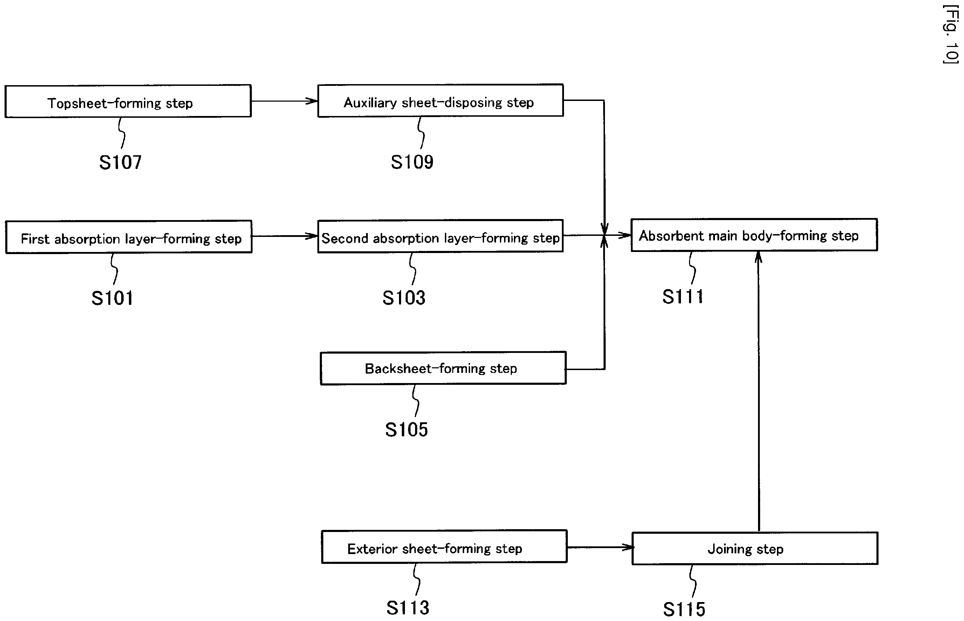

Next, an example of a method of manufacturing the disposable diaper according to at least one embodiment is explained with reference to Fig. 10 through Fig. 12. The manufacturing method explained below is only an example, and the disposable diaper can also be manufactured by other manufacturing methods.

The method of manufacturing the disposable diaper includes, for example, at least a first absorption layer-forming step S101 and a second absorption layer-forming step S103 as the absorber-forming step, a backsheet-forming step S105, a topsheet-forming step S107, an auxiliary sheet-disposing step S109, an absorbent main body-forming step S111, an exterior sheet-forming step S113 of forming an exterior sheet, and a joining step S115 or joining the absorbent main body and the exterior sheet.

The absorber-forming step includes the first absorption layer-forming step S101 of forming the first absorption layer 41, and the second absorption layer-forming step S103 of forming the second absorption layer 42. The absorber-forming step is explained later in detail.

In the absorber 40 formed in the absorber-forming step, the first slit 45, the second slits 46, and the third slits 47 of the first absorption layer 41 are formed so as to be parallel to the conveyance direction MD.

In the backsheet-forming step S105, the sidesheets 60 and the absorber backside covering sheet 30, which configure the backsheet, are formed. In the sidesheet web including two continuous bodies of the sidesheets 60 configuring the leakage-preventing walls, continuous side elastic members, which are two continuous bodies of side elastic members 90 configuring the leakage-preventing walls, are installed. Next, in order to enwrap the continuous side elastic members, both ends of the sidesheet web are folded back, and the folded-back portion is joined by heat seal, for example.

Next, the sidesheet web is split up in the center in the crossing direction CD, that is almost perpendicular to the conveyance direction MD, along the conveyance direction MD.

The absorber backside covering sheet web, which is a continuous body of the absorber backside covering sheet 30, and the continuous second elastic member, which is a continuous body of the second elastic member 48, are pasted (or bonded) on to the sidesheet webs.

The continuous second elastic member is sandwiched and fixed between the absorber backside covering sheet web and the sidesheet web. On the surface where the absorber backside covering sheet web and the sidesheet web are facing each other, and in the continuous second elastic member, an adhesive, such as a hot-melt adhesive is coated according to a predetermined pattern.

In the topsheet-forming step S107, a topsheet web which is a continuous body of the topsheet 10 is formed. In the auxiliary sheet-disposing step S109, an auxiliary sheet web, which is a continuous body of the auxiliary sheet 15, is cut at a predetermined length in the conveyance direction MD to form the auxiliary sheet 15. Also, the auxiliary sheet 15 is pasted intermittently on the topsheet web.

In the absorbent main body-forming step S111, the absorber 40 is pasted on to the absorber backside covering sheet web in which the sidesheet web, which is a continuous body of the sidesheets 60, and the continuous second elastic member, which is a continuous body of the second elastic member 48 have been pasted.

Next, the absorber topside covering sheet web, which is a continuous body of the absorber topside covering sheet 20, is superimposed on the upper surface of the absorber 40, and the absorber topside covering sheet web is pasted on to the absorber 40.

Additionally, the topsheet web on which the auxiliary sheet 15 has been pasted is superimposed on the upper surface of the absorber topside covering sheet web, and the topsheet web is pasted on to the absorber topside covering sheet web.

Next, both sides of the sidesheet web in the crossing direction CD are rolled on the upper surface and fixed to form the leakage-preventing unit. Next, the continuous first elastic member, which is a continuous body of the first elastic member 44, is conveyed in a stretched state along the conveyance direction, and is pasted to the central portion of the absorber backside covering sheet web in the crossing direction CD. Following this, in order to cover the continuous first elastic member, the elastic member covering sheet web, which is a continuous body of the elastic member covering sheet 43, is pasted.

In this way, an absorbent main body web which is a continuous body of the absorbent main body 1A is formed. Also, by cutting the absorbent main body web in the length of a single product, the absorbent main body 1A is formed. At this time, the first elastic member 44, the second elastic member 48, and the side elastic member 90 are in a discontinuous state, and are arranged in a portion where a hot-melt adhesive is coated. Furthermore, each of the webs are cut up in to a single product.

In the exterior sheet-forming step S113, the exterior sheet arranged at the backside of the absorbent main body is formed. Before forming the absorbent main body, the exterior sheet may be formed. In the exterior sheet-forming step, first of all, the exterior topsheet web is split along the conveyance direction MD, and then the continuous body of the foreside exterior topsheet 70F provided in the front waistline region S1, and the continuous body of the backside exterior topsheet 70R provided in the back waistline region S2 are formed.

Similarly, the exterior backsheet web is split along the conveyance direction MD, and then the continuous body of the foreside exterior backsheet 80F provided in the front waistline region S1, and the continuous body of the backside exterior backsheet 80R provided in the back waistline region S2 are formed. These continuous bodies are conveyed along the conveyance direction MD in a spacing in the crossing direction CD.

The continuous waistline elastic member, which is a continuous body of the waistline elastic members 7A, the continuous waist elastic member, which is a continuous body of the waist elastic members 3A, the continuous front leg hole elastic member, which is a continuous body of the front leg hole elastic members 5F, and the continuous back leg hole elastic member, which is a continuous body of the back leg hole elastic members 5R, are installed on the exterior backsheet web while stretching each continuous body in the conveyance direction MD.

At this time, a part of the continuous front leg hole elastic member and the continuous back leg hole elastic member is positioned on the exterior backsheet web, and the other portion is installed so as to be positioned in the spaced-out region, that is, outside the exterior backsheet web.

Next, each component of the exterior topsheet web is superimposed on to the exterior backsheet web, and each elastic member is arranged between the exterior topsheet web and the exterior backsheet web. Next, the continuous front leg hole elastic member and the continuous back leg hole elastic member positioned within the spaced-out region are cut.

By pressing the exterior backsheet web and the exterior topsheet web in this state, a laminated body of the foreside exterior topsheet 70F and the foreside exterior backsheet 80F, and a laminated body of the backside exterior topsheet 70R and the backside exterior backsheet 80R is formed.

The exterior center sheet 100 is pasted to spread across the spaced-out region between the laminated body of the foreside exterior topsheet 70F and the foreside exterior backsheet 80F, and the laminated body of the backside exterior topsheet 70R and the backside exterior backsheet 80R. In this way, the exterior sheet web, which is a continuous body of the exterior sheet, is formed.

Next, the exterior sheet web is cut at a predetermined interval in the conveyance direction MD. By cutting the exterior sheet web along the leg hole elastic members 5R and 5F, a leg hole opening is formed.

In the joining step S 115, the absorbent main body 1A and the exterior sheet are joined. Specifically, the absorbent main body 1A is rotated by 90 degrees, and the absorbent main body 1A is pasted on the exterior sheet web.

Next, the exterior sheet and the absorbent main body are folded along a folding line running along the conveyance direction MD. In such a case, the folding line may either match the center in the crossing direction CD, or may deviate from the center. Also, the front waistline side edges 4 and 4' and the back waistline edges 6 and 6' are joined partially by leaving some interval in the conveyance direction MD, and by cutting along the crossing direction CD in the joining portions, the disposable diaper shown in Fig. 1 can be formed.

In the aforementioned auxiliary sheet-disposing step S109, the auxiliary sheet 15 is arranged such that both ends of the auxiliary sheet 15 in the widthwise direction are positioned near the second curving unit and the third curving unit.

For example, in at least one embodiment, both ends in the widthwise direction of the auxiliary sheet 15 are arranged so as to be positioned at a position almost same as the second slits 46 (preferably at the inner ends from the second slits 46). The rigidity of the region in which the auxiliary sheet 15 is arranged, and the rigidity of the region in which the auxiliary sheet 15 is not arranged (the region outboard of the auxiliary sheet 15 in the widthwise direction) is different. By providing a difference in the rigidity based on the existence of the auxiliary sheet, it becomes easy to curve the absorber with the second curving unit configured by the second slits 46 as the base point.

### (First embodiment)

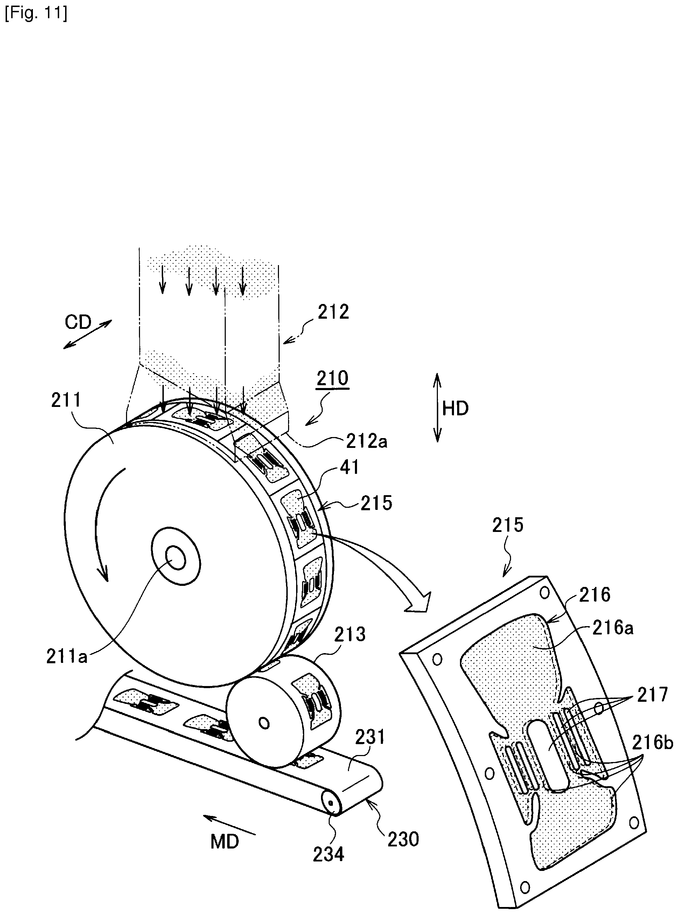

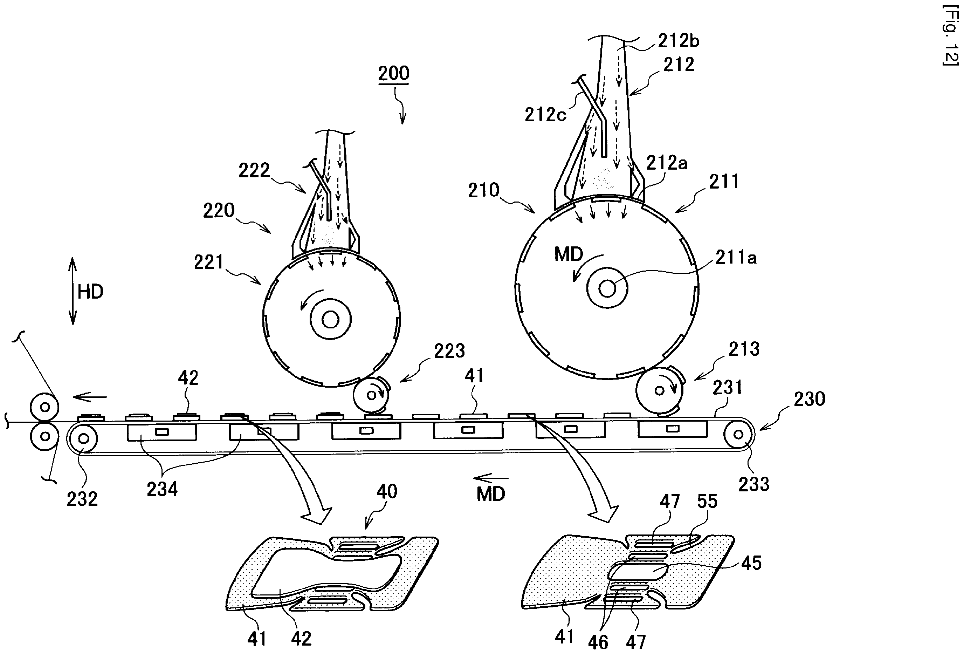

Next, the method of manufacturing the absorber is explained based on Fig. 11 and Fig. 12. Fig. 11 is a partial perspective view of a manufacturing step including a device 200 for manufacturing the absorber 40, and Fig. 12 is a side view of the manufacturing device 200 showing the inside of the manufacturing device 200. In the figure, MD indicates the conveyance direction, CD indicates the crossing (traverse) direction perpendicular to the conveyance direction MD, and HD indicates a vertical direction perpendicular to the directions MD and CD.

The device 200 for manufacturing the absorber includes a first lamination device 210 configured to laminate the absorbent material constituting the first absorption layer 41, a second lamination device 220 configured to laminate the absorbent material constituting the second absorption layer 42, and a conveyor 230.

In the device 200 for manufacturing the absorber 40 according to at least one embodiment, in order to laminate the second absorption layer 42 on the first absorption layer 41 having a curving unit, the second lamination device 220 is arranged downstream in the conveyance direction MD with respect to the first lamination device 210, and then with the help of the conveyor 230, the first absorption layer 41 configured by the first lamination device 210 is conveyed towards the second lamination device 220.

The first lamination device 210 includes a first lamination drum 211, a first absorbent material supply duct 212, and a first delivery roller 213. The second lamination device 220 includes a second lamination drum 221, a second absorbent material supply duct 222, and a second delivery roller 223. In the explanation given below, the configuration that is same for the first lamination device 210 and the second lamination device will be omitted.

The first absorbent material supply duct 212 supplies the pulp fibers and SAP configuring the absorber to the first lamination drum 211. The first absorbent material supply duct 212 is arranged above the first lamination drum 211. A lower opening 212a is formed below the first absorbent material supply duct 212. The lower opening 212a faces the outer circumference of the first lamination drum 211.

A fiber supply port 212b and a polymer supply path 212c is formed in the first absorbent material supply duct 212. The fiber supply port 212b is a portion configured to supply pulp fibers inside the first absorbent material supply duct 212, and is positioned at the top of the first absorbent material supply duct 212.

The polymer supply path 212c is a conduit for supplying highly polymerized absorbent polymer inside the first absorbent material supply duct 212, and has a discharge slot for discharging the highly polymerized absorbent polymer grains towards the first lamination drum 211.

The first lamination drum 211 is hollow cylinder shaped, and is configured to rotate in the conveyance direction MD around a rotation axis 211a. A plurality of forming dies 215 are arranged on the outer circumference of the first lamination drum 211. The forming dies 215 are arranged at the required pitch along the conveyance direction MD.

In the forming dies 215, concave units 216 caving inward in a radial direction of the first lamination drum, and convex units 217 protruding outwards in the radial direction are formed. The concave units 216 have a shape corresponding to a shape of the first absorption layer 41. The convex units 217 have a shape corresponding to the first slit, the second slits, and the third slits. Because the absorbent material is not laminated in the portion where the convex units 217 are formed, the first slit 45, and the like, is formed. The concave units 216 are configured by a base unit 216a, and wall units 216b rising up from the base unit 216a. The convex units 217 protrude outwards in the radial direction from the base unit 216a.

The base unit 216a runs along the outer circumference surface of the first lamination drum 211, and is arranged to extend in the longitudinal direction and widthwise direction of the absorber. The wall units 216b run along the radial direction of the first lamination drum, and are arranged to extend in the inner direction and outer direction of the absorber. The forming dies are a three-dimensional pattern plate.

A plurality of through holes are formed in the wall units 216b and the base unit 216a to communicate between the inside and outside of the first lamination drum 211. Additionally, through holes are formed in the convex units as well. A suction mechanism (not shown in the figure) is arranged on the inner side of the first lamination drum 211. The suction mechanism sucks up the absorbent material inside the concave units at a position facing the first absorber material supply duct 212. Through the suction of the absorbent material supplied inside the concave units 216 of the first lamination drum 211 by the suction mechanism, the first absorption layer is formed while forming the slits. In the first absorption layer thus configured, slits are formed at five locations in the form of curving units.

The absorber 40 formed by the first laminating drum 211 moves along with the rotation of the first laminating drum, and is transferred to the first delivery roller 213. At a position facing the first delivery roller 213, the suction mechanism blows pressurized air towards the concave units 216 of the forming dies 215 via the through holes. Thus, the first absorption layer 41 is transferred to the first delivery roller 213.

The conveyor 230 has a belt member 231, and a drive roller 232 and driven roller 233 configured to suspend the belt member 231. The belt member 231 is air permeable, and is rotated by the drive roller 232 and the driven roller 233. A plurality of through holes (not shown in the figure) are formed in the belt member 231.

Between the drive roller 232 and the driven roller 233, an appropriate number of suction mechanisms 234 are arranged to suck up the air above the belt member 231, via the belt member 231. Although not shown in the figures, in at least one embodiment, the first absorption layer and the second absorption layer may be pressed individually by a press device.

Next, a method of manufacturing an absorbent article by a device 200 for manufacturing the absorber, which has thus been configured, is explained. First of all, the absorbent material supply step S1011 is performed as the first absorption layer-forming step S101. Specifically, the absorbent material constituting the first absorption layer 41 is laminated inside the concave units 216 of the first lamination drum 211.

Next, the suction step S1012 is performed as the first absorption layer-forming step S101. Specifically, the air inside the concave units 216 is sucked up via through holes, with the help of the suction mechanism of the first lamination drum 211. By sucking up the absorbent material with the suction mechanism, the first absorption layer 41 can be formed while forming the first slit 45, the second slits 46, and the third slits 47.

The first absorption layer 41 formed by laminating by the first lamination drum 211 is transferred to the first delivery roller 213, following which the first absorption layer is transferred on to the conveyor 230 from the first delivery roller 213. The first absorption layer 41 is transferred on to the conveyor 230 by the suction mechanism of the first lamination drum and the suction mechanism of the conveyor. The first absorption layer 41 is arranged on the belt member 231 of the conveyor 230, and is conveyed towards the second lamination device 220.

Next, the second absorption layer 42 is formed in the same way as the first absorption layer 41, by the second lamination device 220. Convex units are not provided in the concave units of the forming dies by which the second absorption layer 42 is formed. As a result, the configuration is such that the slits configuring the curving units are not formed in the second absorption layer 42. Then, the second absorption layer 42 formed by the second lamination drum 221 is laminated on top of the first absorption layer 41 via the second delivery roller 223.

At this time, the second absorption layer 42 is arranged by covering some of the slits (for example, the first slit 45) of the first slit 45, the second slits 46, and the third slits 47 formed in the first absorption layer with the second absorption layer 42, and leaving the other slits (for example, the third slits 47) open without being covered by the second absorption layer 42.

Next, the first absorption layer 41 and the second absorption layer 42 are joined together to form the absorber 40. The absorber is joined with a sheet material, such as the topsheet, in the course of being conveyed by a conveyor, as described above, and the disposable diaper is thus manufactured (although not shown in the figures).

In the absorber 40 thus manufactured, the first absorption layer 41 positioned down is sucked in uniformly by the suction mechanism of the conveyor 230 while being conveyed by the conveyor 230. On the other hand, because the second absorption layer 42 positioned on the top is sucked in via the first absorption layer 41, suction is not uniform. Therefore, for example, if slits are formed in the second absorption layer 42, due to non-uniform suction by the suction mechanism, the absorber between the slits might get torn or folded.

However, because the first absorption layer 41 that is positioned at the lower side is sucked in uniformly by the suction mechanism of the conveyor 230, even when slits are formed, it becomes difficult for the absorber to tear off or to get deformed. Therefore, the first absorption layer 41 in which slits are formed is arranged at the lower side in at least one embodiment.

On the other hand, because the slits that act as the curving units are not provided in the second absorption layer 42 (i.e., the second absorption layer 42 is free of slits), there are no portions with a narrow width, as between the slits of the first absorption layer 41. Therefore, the absorber does not get torn or twist easily during the transfer of the second absorption layer 42, and stable transfer process can be performed, which can prevent deformation of the absorber.

Furthermore, when slits are provided in both the first absorption layer 41 and the second absorption layer 42, the position of the absorber may deviate when the first absorption layer 41 and the second absorption layer 42 are superimposed. For example, if the position deviates in the widthwise direction, the width of the pair of slits arranged on the left and right becomes narrow, regular deformation might not occur, and an absorber with crosswise unbalance is produced, which may exert a bad influence on absorbency and the feeling of comfort at the time of wearing the absorbent article.

Additionally, in the crotch region S3 in which slits are formed in the first absorption layer 41, the outer ends 42W of the second absorption layer 42 in the widthwise direction are arranged by overlapping the second slits 46. Therefore, due to the difference in the rigidity in the crotch region S3 between the inner side and the outer side of second slits 46 in the widthwise direction, it becomes easy for the absorber 40 to bend, and the deformation at the time of wearing the absorbent article can be stabilized, and the feeling of discomfort can be reduced.

### (Second embodiment)

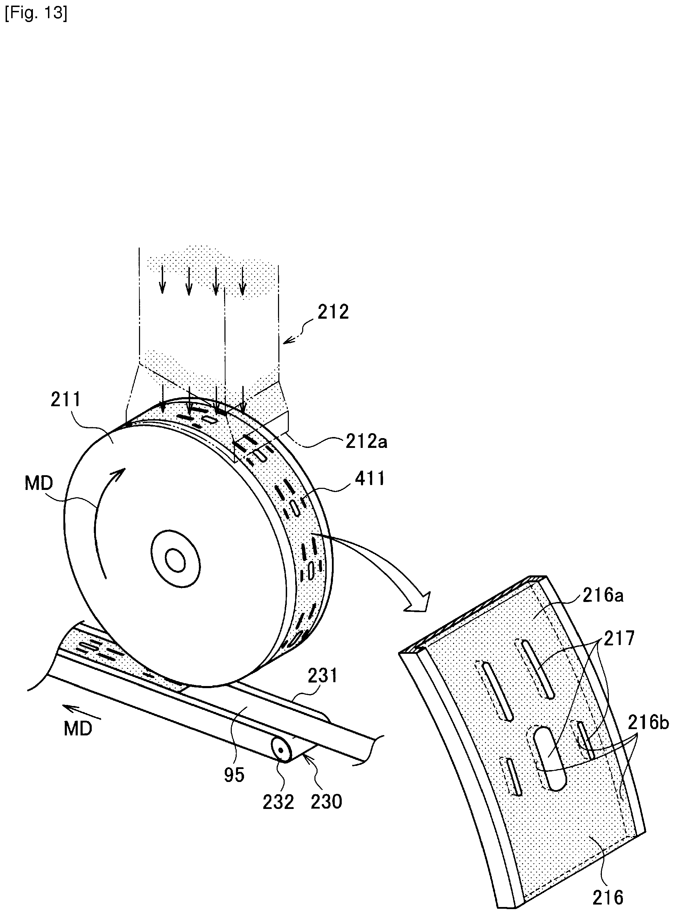

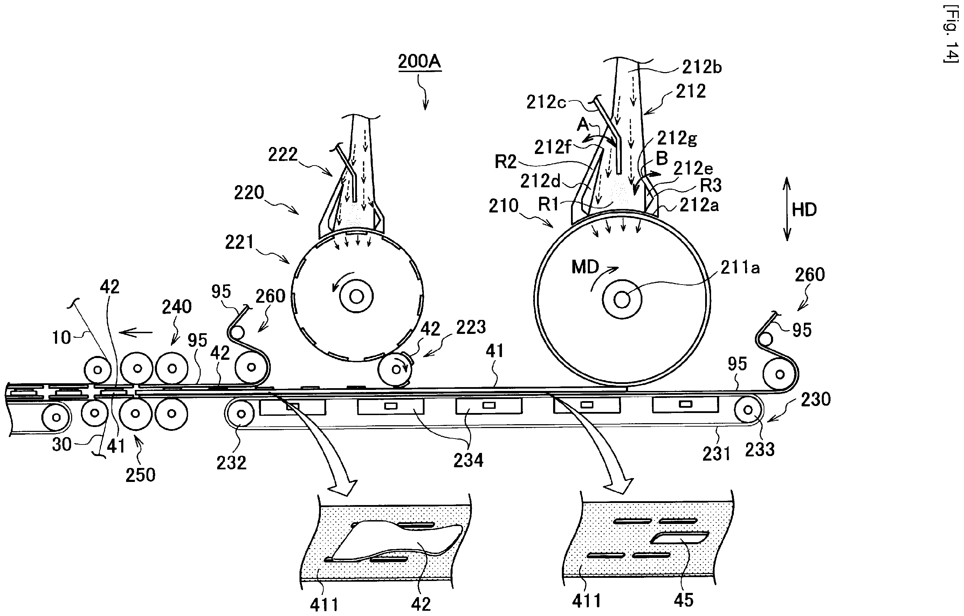

Next, a method of manufacturing the absorbent article according to a second embodiment is explained in detail with reference to Fig. 13 and Fig. 14. Fig. 13 is a partial perspective view of a step including a device 200A for manufacturing the absorber 40, and Fig. 14 is a side view of the manufacturing device 200A showing the inside of the manufacturing device 200A. In the explanation of the second embodiment, the same symbols have been used for the configuration that is the same as the first embodiment, and the explanation has been omitted.

The device 200A for manufacturing the absorber according to the second embodiment includes the first lamination device 210 configured to laminate the absorbent material constituting the first absorption layer 41, the second lamination device 220 configured to laminate the absorbent material constituting the second absorption layer 42, the conveyor 230, a press device 240, and a cutting device 250.

The first lamination device 210 includes the first lamination drum 211, and the first absorbent material supply duct 212. The second lamination device 220 includes a second lamination drum 221, a second absorbent material supply duct 222, and a second delivery roller 223.

In the first lamination drum 211 according to the second embodiment, a single concave unit 216 for forming the first absorption layer 41 is formed in the circumferential face of the first lamination drum 211. Therefore, the first absorber is formed as a continuous body by the first lamination drum 211.

Furthermore, first and second tissue supply devices 260 configured to supply a tissue layer 95 for wrapping the first absorption layer 41 and the second absorption layer 42 are arranged on the upstream side of the first lamination device 210 and on the downstream side of the second lamination device 220, respectively.

The continuous body formed by the first lamination drum 211 is transferred on to the continuous tissue layer 95 supplied by the first tissue supply device 260 on the upstream side of the first lamination device 210. In at least one embodiment, a hot-melt adhesive is applied beforehand on the tissue layer 95, and the first absorption layer 41 and the tissue layer 95 are joined via the hot-melt adhesive.

Furthermore, similar to the first lamination drum according to the first embodiment, if the absorption layer is formed intermittently by concave units arranged intermittently, the suction of the absorbent material in the concave units is not uniform, and the lamination distribution of the absorbent material might not be stable. However, by forming the absorption layer continuously by a single (or continuous) concave unit, the lamination distribution of the absorbent material can be stabilized.

Further downstream of the second tissue supply device 260 on the downstream side of the second lamination device, the press device 240, and the cutting device 250 are arranged. The press device 240 presses the first absorption layer 41 and the second absorption layer 42 arranged between the tissue layers 95 by press rollers. Although not shown in the figures, the thickness of the first absorption layer 41 and the second absorption layer 42 is reduced by pressing. Furthermore, when performing the pressing operation, by arranging the tissue layers 95 between the press rollers and the absorber, the adherence of the pulp fibers and SAP on to the press rollers can be prevented.

Although it has been described that one tissue layer 95 is arranged on the lower side of the first absorption layer 41 and another tissue layer 95 is arranged on the upper side of the second absorption layer 42, however, for example, either one of the tissue layers 95 may be arranged, and by wrapping the first absorption layer 41 and the second absorption layer 42 by the tissue layer 95, the first absorption layer 41 and the second absorption layer 42 may be wrapped.

Furthermore, by supplying a sheet, such as the tissue layer 95, on to several concave units of forming dies formed on the outer circumference surface of the second lamination drum 221, before the absorbent material supply step of the second lamination drum 221, the absorbent material constituting the second absorption layer is deposited on the sheet, and an absorber including the sheet between the first absorption layer and the second absorption layer can be obtained.

By applying a hot-melt adhesive beforehand on the upper surface and lower surface of the sheet, such as the tissue layer, and then pasting each absorption layer and the sheet, the separation of the absorption layer and the sheet can be prevented, and an absorber that does not get deformed easily can be obtained.

The cutting device 250 cuts the first absorption layer 41 and the second absorption layer 42 on which pressing has been performed, in to single pieces.

Next, a method of manufacturing an absorbent article by the device 200A for manufacturing the absorber, which has thus been configured, is explained. The absorbent material supply step S1011 is performed as the first absorption layer-forming step S101. Specifically, the absorbent material constituting the first absorption layer 41 is laminated inside the concave units 216 of the first lamination drum 211.

Next, the suction step S1012 is performed as the first absorption layer-forming step S101. Specifically, the air inside the concave units is sucked up via through holes, with the help of the suction mechanism of the first lamination drum 211. By sucking up the absorbent material with the suction mechanism, the continuous body 411 of the first absorption layer 41 can be formed while forming the first slit 45, the second slits 46, and the third slits 47.

The continuous body of the first absorption layer 41 formed by the first lamination drum 211 is transferred on to the tissue layer 95 of the conveyor 230. The continuous body 411 of the first absorption layer 41 is conveyed towards the second lamination device 220 by the conveyor 230.

Next, the second absorption layer 42 is formed by the second lamination device 220. Same as in the first embodiment, the second lamination device 220 forms individual absorbent articles. The second absorption layer 42 formed by the second lamination drum is laminated on top of the continuous body 411 of the first absorption layer 41, via the delivery roller 223.

Next, the tissue layer 95 is supplied on top of the second absorption layer 42, by the tissue supply device 260. While sandwiching the first absorption layer and the second absorption layer by the tissue layer 95, the first absorption layer 41 and the second absorption layer 42 are conveyed towards the press device 240. After pressing the tissue layer 95, the first absorption layer 41, and the second absorption layer 42 by the press device, cutting in one piece is performed by the cutting device 250, and the absorber 40 is formed.

In the second embodiment, a continuous body of the first absorption layer is formed, and then the second absorption layer is formed in pieces, however, the second absorption layer may also be formed as a continuous body, and then cut along with the first absorption layer, or cut separately from the first absorption layer, to form the absorber 40.

A first movable partition wall 212d positioned on the upstream side in the conveyance direction MD, and a second movable partition wall 212e positioned on the downstream side are formed inside the first absorbent material supply duct 212 according to the second embodiment. The first movable partition wall 212d and the second movable partition wall 212e are configured such that the respective upper ends 212f and 212g can turn in the direction of the dual-head arrows A and B, around the axis extending in the crossing direction CD (not shown in the figure)

The first movable partition wall 212d and the second movable partition wall 212e fulfill two states, that is, the first state in which the upper ends 212f and 212g are alienated from the inner wall of the first absorbent material supply duct 212, and the second state in which the upper ends 212f and 212g are in contact with the inner wall of the first absorbent material supply duct 212.

In the first state, the pulp fibers supplied to the fiber supply port 212b are dropped while being dispersed from the main route R1 between the first movable partition wall 212d and the second movable partition wall 212e, the upstream route R2 at the upstream side from the first movable partition wall 212d, and the downstream route R3 at the downstream side from the second movable partition wall 212e, and then supplied to the outer circumference surface of the first lamination drum 211. The highly polymerized absorbent polymer is supplied inside all the forming dies in a state of being mixed with the pulp fibers, preferably being mixed uniformly.

On the other hand, in the second state, the pulp fibers supplied from the fiber supply port 212b are not supplied to the upstream route R2 and the downstream route R3, but are supplied only to the main route R1 between the first movable partition wall 212d and the second movable partition wall 212e.

Furthermore, the polymer supply path 212c is configured to be able to move in the vertical direction HD. In a state in which the polymer supply path 212c comes close to the first lamination drum 211, the highly polymerized absorbent polymer is not supplied to the upstream route R2 and the downstream route R3, and the amount of the highly polymerized absorbent polymer included in the top layer portion of the front and back sides of the absorber 40 reduces.

In this way, by moving the first movable partition wall 212d and the second movable partition wall 212e, and moving the position of the polymer supply path 212c in the vertical direction, the amount of the highly polymerized absorbent polymer included in the top layer portion of the front and back sides of the absorber 40 can be reduced by adjusting the supply path of the highly polymerized absorbent polymer. Therefore, an absorbent fiber layer with a small ratio of highly polymerized absorbent polymer can be provided in the top layer portion of the top and back sides of the absorber 40.

### (Modifications)

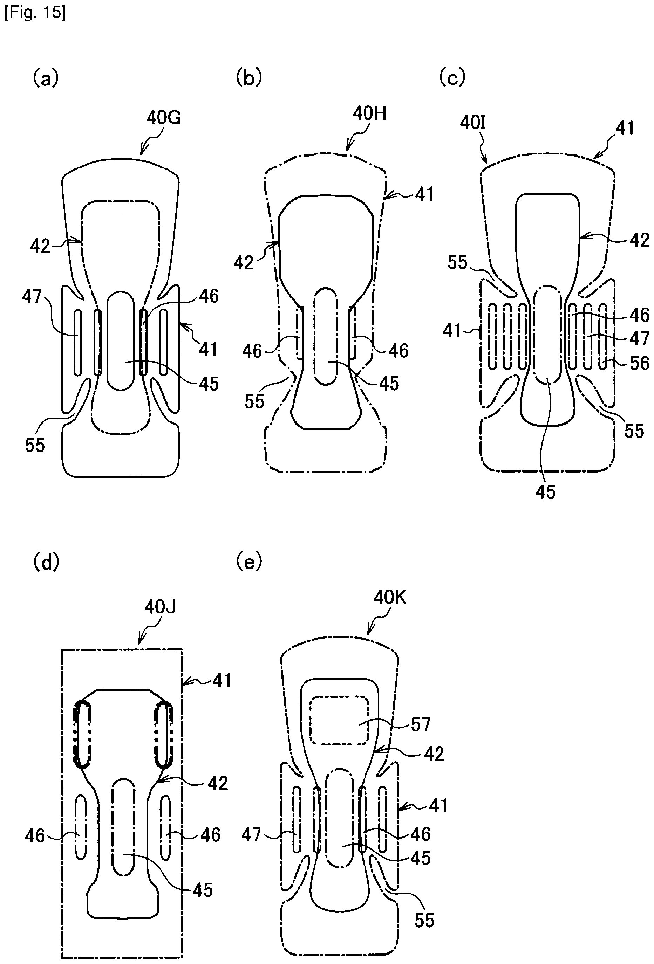

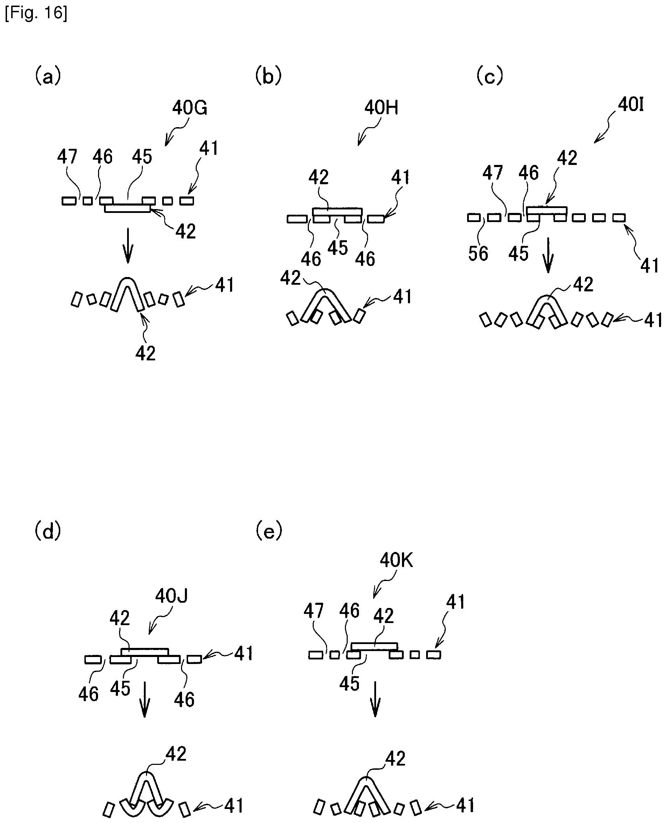

Next, the configuration of the absorbers 40G, 40H, 40I, 40J, and 40K of the disposable diaper according to the first modification through fifth modification is explained with reference to drawings. Note that the same symbols will be used for the same portions as the disposable diaper 1 according to the aforementioned embodiments, and the differences will be mainly explained. Fig. 15 is a plan view of an absorber of the disposable diaper according to the first modification through fifth modification. Fig. 16 is a schematic cross-sectional view of the state of an absorber according to the modification prior to its deformation, and after deformation. Fig. 16 shows the cross section of the narrow region S32 of the absorber. In Fig. 15, the absorption layer positioned at the upper side is shown by a solid line and the absorption layer positioned at the lower side is shown by a chain double-dashed line.

As shown in Fig. 15(a), in an absorber 40G according to the first modification, the arrangement of the first absorption layer 41 and the second absorption layer 42 is reverse as compared to the absorber 40. That is, the first absorption layer 41 in which the first slit 45, the second slits 46, and the third slits 47, and recessed units 55 are formed is arranged at the upper side (topsheet side), and the second absorption layer 42, which is in the shape of an hourglass and where slits are not formed is arranged at the lower side (backsheet side).

The disposable diaper according to the earlier embodiment is formed by joining the first absorption layer positioned at the lower side and the second absorption layer positioned at the upper side, followed by joining the topsheet from the second absorption layer side positioned at the upper side, and then joining the backsheet from the first absorption layer side positioned at the lower side, however, for example, by joining to the absorber with a reverse arrangement of the topsheet and the backsheet, a disposable diaper in which the first absorption layer is provided at the topsheet side can be formed.

Specifically, by joining the first absorption layer positioned at the lower side and the second absorption layer positioned at the upper side, followed by joining the backsheet from the second absorption layer side positioned at the upper side, and then joining the topsheet from the first absorption layer side positioned at the lower side, and then joining the exterior sheet at the backsheet side, a disposable diaper in which the first absorption layer is provided at the topsheet side can be formed.

As shown in Fig. 15 (b), in an absorber 40H according to the second modification, the first absorption layer is positioned at the lower side, and the second absorption layer is positioned at the upper side. The recessed units 55 of the first absorption layer are formed only anterior to the second slits 46 formed in the crotch region, and are not formed posterior to the second slits 46. Three slits are formed in the first absorption layer.

As shown in Fig. 15 (c), in an absorber 40I according to the third modification, the outer ends in the widthwise direction of the second absorption layer are arranged near the inner ends in the widthwise direction of the second slits 46. Furthermore, the fourth slits 56 are formed outboard of the third slit in the widthwise direction.

As shown in Fig. 15 (d), in an absorber 40J according to the fourth modification, the first absorption layer in which the slits are formed is arranged at the lower side, and the second absorption layer is positioned at the upper side. The first absorption layer according to the fourth modification has a rectangular shape in the plan view, and is manufactured by the manufacturing method according to the second embodiment.

The absorbers according to the other modifications can also be manufactured by the manufacturing method according to the second embodiment. For example, by cutting along the outline of the first or second absorption layer at the time of cutting the continuous body, an absorber according to various modifications can be manufactured.

As shown in Fig. 15 (e), an absorber 40K according to the fifth modification is an example of addition of yet another absorption layer to the absorber 40 according to the first embodiment. A top absorption layer 57 in which a curving unit is not formed is laminated at the upper side of the second absorption layer. In Fig. 15(e), the top absorption layer 57 is indicated by a chain double-dashed line.

In the top absorption layer 57, as compared to other absorption layers, the configuration ratio of highly polymerized absorbent polymer is high. The top absorption layer 57 is arranged in the back waistline region S2 that is in contact with the hip of the wearer. By providing the top absorption layer 57 in the portion corresponding to the hip portion of the wearer, the amount of reverse flow of the excretions towards the hip portion is reduced, and the comfort of the skin can be improved.

### (Other Embodiments)

As mentioned above, although several embodiments of the present invention have been disclosed, the descriptions and drawings that form a part of this disclosure are not to be considered as limitation to the present invention. From this disclosure, a variety of alternate embodiments, examples, and applicable techniques will become apparent to one ordinarily skilled in the art.

For example, in the above embodiments, a pants-type disposable diaper was explained, however, the present invention is not limited thereto, and can be applied to an open-type disposable diaper, incontinence pad, sanitary napkin, and other types of absorptive products.

In the aforementioned embodiments, the absorber is configured to be curved by using slits, elastic materials, and/or boundary portions where the rigidity changes, however, the absorber can also be configured to be curved by reducing the thickness of the absorber and by performing embossing in the absorber.

Furthermore, in the absorbent article according to the described embodiments, side end elastic members 49 are provided, however, the side end elastic members 49 need not necessarily be provided. Furthermore, in order to prevent side leakage from the crotch, the leakage-preventing walls have been described to include an upright unit (side elastic members 90), but the leakage-preventing walls need not necessarily have the side elastic members 90.

In the described embodiments, the absorber 40 has a double-layered structure including the first absorption layer 41 and the second absorption layer 42, but the absorber 40 of the worn article according to further embodiments of the present invention can have three or more layers. Even when the absorber 40 is configured by a plurality of absorption layers, only the first absorption layer has curving units in the crotch region, and the other absorption layers may be configured so as not to include curving units in the crotch region. Therefore, the absorption layers other than the first absorption layer may have a curving unit in a region other than the crotch region.

The forming die of the absorber in the described embodiments has a 3D mesh structure in which through holes are formed in the base unit and the wall units, however, for example, the forming die may have a 2D-mesh structure in which through holes are formed only in the base unit.

However, by using a forming die having a 3D-mesh structure, a layer of only pulp fibers can be provided at the sides of the slits formed in the absorption layed By providing a layer of only pulp fibers at the sides of the slits, in an absorber in which pulp and granular SAP are mixed together, the migration of granular SAP into the slits during manufacturing and usage can be prevented. Therefore, the loss at the time of manufacturing can be reduced, and the folding effect and diffusion and absorption effect of the excretions, which are functions of the slits, can be exhibited.

Furthermore, there is no limit on the number of slits that can be formed in the absorber. Specifically, in the crotch region S3 of the absorber, the slits may be arranged symmetrically with respect to the virtual central line EL dividing the width of the absorber in the widthwise direction into two, and extending in the longitudinal direction, or seven or more (or less) slits may be arranged.

In the absorbent article according to the described embodiments, a backsheet and an exterior sheet are arranged in the outer direction of the absorber 40, however, the absorbent article is not limited to this configuration, and only an exterior sheet, or only a backsheet may be arranged in the outer direction of the absorber 40.

As described above, needless to say, the present invention includes various embodiments and the like not described here. Therefore, the scope of the present invention is to be defined only by the inventive specific matter according to the adequate claims from the above description.

## Claims

1. A method of manufacturing an absorbent article including an absorber (40) having a longitudinal direction, a widthwise direction perpendicular to the longitudinal direction, an inner direction for facing a wearer, and an outer direction opposite to the inner direction, wherein a curving unit that allows the absorber (40) to curve in the inner direction or the outer direction is formed in the absorber, said method comprising:
a first absorption layer-forming step of forming a first absorption layer (41) configuring the absorber (40); and
a second absorption layer-forming step of forming a second absorption layer (42) configuring the absorber (40), and laminated on to the first absorption layer (41), wherein
in the first absorption layer-forming step, the first absorption layer (41) is formed with the curving unit in symmetry with respect to a virtual central line dividing a width of the absorber (40) in the widthwise direction into two, and extending in the longitudinal direction, and
in the second absorption layer-forming step, the second absorption layer (42) is formed without providing the curving unit in a crotch region (S3) that is adapted to be in contact with a crotch of the wearer, wherein the curving unit is configured by
a slit formed in the first absorption layer (41), and
wherein in the first absorption layer-forming step,
a first curving unit including a first slit (45) and extending in the longitudinal direction is formed in the center of the crotch region (S3) in the widthwise direction, and
a second curving unit including a pair of second slits (46) and extending in the longitudinal direction is formed outboard of the first curving unit in the widthwise direction, and
a third curving unit including a pair of third slits (47) and extending in the longitudinal direction is formed outboard of the second curving unit in the widthwise direction.

2. The method according to claim 1, wherein in the second absorption layer-forming step,
the second absorption layer (42) is laminated and formed such that a region of the curving unit formed in the first absorption layer (41) is covered with the second absorption layer (42), and
another region of the curving unit is left open without being covered by the second absorption layer (42).

3. The method according to claims 1 or 2, wherein in the second absorption layer-forming step, the second absorption layer is formed such that
the first curving unit formed in the first absorption layer (41) is covered with the second absorption layer (42), and
the second curving unit is not covered completely by the second absorption layer (42), but a part of the second curving unit is left open.

4. The method according to claim 1, wherein the first slit is formed in the center of
the first absorption layer (41) in the widthwise direction,
the pair of second slits is positioned outboard of the first slit in the widthwise direction, and
the pair of third slits is positioned outboard of the pair of second slits in the widthwise direction.

5. The method according to any one of claim 1 through claim 4, further comprising
an elastic member-disposing step of providing an elastic member (44) in a stretched state in the longitudinal direction so as to overlap the curving unit in a thickness direction of the absorber (40).

6. The method according to any one of claim 1 through claim 5, wherein at least one of the first absorption layer-forming step and the second absorption layer-forming step includes
an absorbent material supply step of supplying an absorbent material configuring the absorber (40), to a forming die which includes
a concave unit corresponding to a shape of the first absorption layer (41) or
the second absorption layer (42), and
a through hole that enables suction of the air inside the concave unit, and
a suction step of forming the first absorption layer (41) or the second absorption layer (42) by sucking in the absorbent material.

7. The method according to any one of claim 1 through claim 6, wherein at least one of the first absorption layer-forming step and the second absorption layer-forming step includes
an absorbent material supply step of supplying an absorbent material configuring the absorber (40), to a forming die which includes
a concave unit corresponding to a shape of a continuous arrangement of the first absorption layer (41) or the second absorption layer (42), and
a through hole that enables suction of the air inside the concave unit,
a suction step of forming a continuous body of the first absorption layer (41) or the second absorption layer (42), by sucking in the absorbent material,
and
a cutting step of forming the first absorption layer (41) or the second absorption layer (42), by cutting the continuous body along the widthwise direction.

8. The method according to claim 7, wherein
the concave unit of the forming die includes a base unit (216a) and a wall unit (216b) rising up from the base unit (216a), and the through hole is formed in the wall unit (216b), and
in the suction step, either the first absorption layer (41) or the second absorption layer (42) is formed by sucking in the absorbent material via at least the through hole in the wall unit (216b).

9. The method according to any one of claim 1 through claim 8, further comprising:
a topsheet-forming step of forming a topsheet (10);
a backsheet-forming step of forming a backsheet;
an auxiliary sheet-disposing step of disposing a auxiliary sheet (15) between the topsheet (10) and the absorber (40); and
a joining step of disposing the absorber (40) between the auxiliary sheet (15) and the backsheet, and then joining each of the topsheet (10), the auxiliary sheet (15), the backsheet, and the absorber (40) into one unit, wherein
in the auxiliary sheet-disposing step, the auxiliary sheet (15) is arranged such that both ends of the auxiliary sheet in the widthwise direction are positioned near the curving unit.

10. The method according to any one of claim 1 through claim 9, further comprising:
a step of supplying a sheet between the first absorption layer (41) and the second absorption layer (42) before the first absorption layer (41) and the second absorption layer (42) are laminated.

11. The method according to claim 6 through claim 9, further comprising:
a step of supplying a sheet to cover the concave unit for forming the first absorption layer or the second absorption layer before the absorbent material supply step, such that the absorbent material constituting the second absorption layer (42) is deposited on the sheet.

12. The method according to claim 11, further comprising:
a step of applying hot-melt adhesive on opposite surfaces of the sheet to bond the first and second absorption layers (41, 42) to the opposite surfaces of the sheet.

13. The method according to any one of claim 1 through claim 12, wherein the second absorption layer (42) is formed to be free of slits.

## Patentansprüche

1. Verfahren zur Herstellung eines saugfähigen Artikels, der einen Absorber (40) mit einer longitudinalen Richtung, einer Breitenrichtung senkrecht zu der longitudinalen Richtung, einer einem Träger zugewandten Innenrichtung, und einer Außenrichtung gegenüber der Innenrichtung umfasst, wobei ein gekrümmtes Element, das es dem Absorber (40) erlaubt sich in der Innenrichtung oder der Außenrichtung zu krümmen, in dem Absorber ausgebildet ist, das Verfahren umfassend:
einen ersten absorptionsschichtbildenden Schritt des Ausbildens einer ersten Absorptionsschicht (41), die den Absorber (40) bestimmt; und
einen zweiten absorptionsschichtbildenden Schritt des Ausbildens einer zweiten Absorptionsschicht (42), die den Absorber (40) bestimmt, und auf die erste Absorptionsschicht (41) laminiert, wobei
in dem ersten absorptionsschichtbildenden Schritt die erste Absorptionsschicht (41) mit dem gekrümmten Element symmetrisch unter Berücksichtigung einer virtuellen, zentralen Linie, die eine Breite des Absorbers (40) in Breiternrichtung in zwei teilt, und sich in longitudinaler Richtung ersteckt, ausgebildet ist, und
in dem zweiten absoptionschichtbildenden Schritt die zweite Absorptionschicht (42), ohne das gekrümmte Element in einer Schrittregion (S3), die angepasst ist um in Kontakt mit einem Schritt des Trägers zu sein, aufzuweisen ausgebildet ist,
wobei das gekrümmte Element konfiguriert ist durch
einen in der ersten Absorptionschicht (41) ausgebildeten Schlitz, und
wobei in dem ersten absorptionschichtbildenden Schritt,
ein erstes gekrümmtes Element, das einen ersten Schlitz (45) umfasst und sich in der longitudinalen Richtung erstreckt, im Zentrum des Schrittbereichs (S3) in der Breitenrichtung ausgebildet ist, und
ein zweites gekrümmtes Element, das ein Paar zweite Schlitze (46) umfasst und sich in der longitudinalen Richtung erstreckt, außerhalb des ersten gekrümmten Elements in der Breitenrichtung ausgebildet ist, und
ein drittes gekrümmtes Element, das ein Paar dritte Schlitze (47) umfasst und sich in der longitudinaler Richtung erstreckt, außerhalb des zweiten gekrümmten Elements in der Breitenrichtung ausgebildet ist.

2. Das Verfahren gemäß Anspruch 1, wobei in dem zweiten absorptionsschichtbildenden Schritt,
die zweite Absorptionsschicht (42) so laminiert und ausgebildet ist, dass ein Bereich des gekrümmten Elements in der ersten Absorptionsschicht (41) mit der zweiten Absorptionsschicht (42) bedeckt ist, und
ein anderer Bereich des gekrümmten Elements offen gelassen ist ohne von der zweiten Absorptionsschicht (42) bedeckt zu sein.

3. Das Verfahren gemäß der Ansprüche 1 oder 2, wobei in dem zweiten absorptionsschichtbildenden Schritt, die zweite Absorptionsschicht so ausgebildet ist, dass das in der ersten Absorptionsschicht (41) ausgebildete erste gekrümmte Element mit der zweiten Absorptionsschicht (42) bedeckt ist, und
das zweite gekrümmte Element nicht vollständig von der zweiten Absorptionsschicht (42) bedeckt ist, sondern ein Teil des zweiten gekrümmten Elements offen gelassen ist.

4. Das Verfahren gemäß Anspruch 1, wobei der erste Schlitz im Zentrum von
der ersten Absorptionsschicht (41) in der Breitenrichtung,
dem Paar zweite Schlitze, das außerhalb des ersten Schlitzes in der Breitenrichtung positioniert ist, und
dem Paar dritte Schlitze, das außerhalb des Paares zweiter Schlitze in der Breitenrichtung positioniert ist, ausgebildet ist.

5. Das Verfahren gemäß einem der Ansprüche 1 bis 4, zusätzlich
einen ein elastisches Teil anordnenden Schritt des Bereitstellens eines elastischen Teils (44) in einem gedehnten Zustand in der longitudinalen Richtung, um das gekrümmte Element in einer Dickenrichtung des Absorbers (40) zu überlappen, umfassend.

6. Das Verfahren gemäß einem der Ansprüche 1 bis 5, wobei wenigstens einer von dem ersten absorptionsschichtbildenden Schritt und dem zweiten absorptionsschichtbildenden Schritt
einen saugfähiges Material Zufuhrschritt des Zuführens eines saugfähigen Materials, das den Absorber (40) konfiguriert, zu einem Formwerkzeug, das
ein konkaves Element, das einer Form der ersten Absorptionsschicht (41) oder der zweiten Absorptionsschicht (42) entspricht umfasst, und
ein Durchgangsloch, das Ansaugen der Luft innerhalb des konkaven Elements ermöglicht, und
einen Ansaugschritt des Ausbildens der ersten Absorptionsschicht (41) oder der zweiten Absorptionsschicht (42) durch Ansaugen des saugfähigen Materials umfasst.

7. Das Verfahren gemäß einem der Ansprüche 1 bis 6, wobei wenigstens einer des ersten absorptionsschichtbildenden Schritts und des zweiten absorptionsschichtbildenden Schritts
einen saugfähiges Material Zufuhrschritt des Zuführens eines saugfähigen Materials, das den Absorber (40) konfiguriert, zu einem Formwerkzeug, das
ein konkaves Element, das einer Form einer kontinuierlichen Anordnung der ersten Absorptionsschicht (41) oder der zweiten Absorptionsschicht (42) entspricht umfasst, und
ein Durchgangsloch, das Ansaugen der Luft innerhalb des konkaven Elements ermöglicht,
einen Ansaugschritt des Ausbildens eines kontinuierlichen Körpers der ersten Absorptionsschicht (41) oder der zweiten Absorptionsschicht (42) durch Ansaugen des saugfähigen Materials, und
einen Schnittschritt des Ausbildens der ersten Absorptionsschicht (41) oder der zweiten Absorptionsschicht (42) durch Schneiden des kontinuierlichen Körpers entlang der Breitenrichtung, umfasst.

8. Das Verfahren gemäß Anspruch 7, wobei
das konkave Element des Formwerkzeugs ein Grundelement (216a) und ein Wandelement (216b), das sich von dem Grundelement (216a) erhebt, umfasst und das Durchgangsloch in dem Wandelement (216b) ausgebildet ist, und
in dem Ansaugschritt, entweder die erste Absorptionsschicht (41) oder die zweite Absorptionsschicht (42) durch Ansaugen des saugfähigen Materials über wenigstens das Durchgangsloch in dem Wandelement (216b) ausgebildet ist.

9. Das Verfahren gemäß einem der Ansprüche 1 bis 8, zusätzlich umfassend:
einen oberlagebildenden Schritt des Ausbildens einer Oberlage (10);
einen unterlagebildenden Schritt des Ausbildens einer Unterlage;
einen hilfslageanordnenden Schritt des Anordnens einer Hilfslage (15) zwischen der Oberlage (10) und dem Absorber (40); und
einen Verbindungsschritt des Anordnens des Absorbers (40) zwischen der Hilfslage (15) und der Unterlage, und dann des Verbindens jede der Oberschicht (10), der Hilfsschicht (15), der Unterschicht, und des Absorbers (40) in ein Element, wobei in dem hilfslageanordnenden Schritt, die Hilfslage (15) so angeordnet ist, dass beide Enden der Hilfslage in Breitenrichtung nahe des gekrümmten Elements angeordnet sind.

10. Das Verfahren gemäß einem der Ansprüche 1 bis 9, zusätzlich umfassend:
einen Schritt des Zuführens einer Lage zwischen der ersten Absorptionsschicht (41) und der zweiten Absorptionsschicht (42) bevor die erste Absorptionsschicht (41) und die zweite Absorptionsschicht (42) laminiert werden.

11. Das Verfahren gemäß der Ansprüche 6 bis 9, zusätzlich umfassend:
einen Schritt des Zuführens einer Lage zum Bedecken des konkaven Elements um die erste Absorptionsschicht oder die zweite Absorptionsschicht vor dem saugfähigen Material Zufuhrschritt auszubilden, so dass das saugfähige Material, welches die zweite Absorptionsschicht (42) darstellt, auf die Lage aufgetragen wird.

12. Das Verfahren gemäß Anspruch 11, zusätzlich umfassend:
einen Schritt des Aufbringens heißen-geschmolzenen Klebers auf gegenüberliegende Oberflächen der Lage um die erste und die zweite Absorptionsschicht (41, 42) mit den gegenüberliegenden Oberflächen der Lage zu verbinden.

13. Das Verfahren gemäß einem der Ansprüche 1 bis 12, wobei die zweite Absorptionsschicht (42) schlitzfrei ausgebildet ist.

## Revendications

1. Procédé de fabrication d'un article absorbant incluant un absorbeur (40) présentant une direction longitudinale, une direction de la largeur perpendiculaire à la direction longitudinale, une direction intérieure pour faire face à un porteur, et une direction extérieure opposée à la direction intérieure, dans lequel une unité de courbage qui permet à l'absorbeur (40) de se courber dans la direction intérieure ou la direction extérieure est formée dans l'absorbeur, ledit procédé comprenant :
une étape de formation de première couche d'absorption de formation d'une première couche d'absorption (41) configurant l'absorbeur (40) ; et
une étape de formation de seconde couche d'absorption de formation d'une seconde couche d'absorption (42) configurant l'absorbeur (40), et disposée sur la première couche d'absorption (41), dans lequel
dans l'étape de formation de première couche d'absorption, la première couche d'absorption (41) est formée avec l'unité de courbage en symétrie par rapport à une ligne centrale virtuelle divisant une largeur de l'absorbeur (40) dans la direction de la largeur en deux, et s'étendant dans la direction longitudinale, et
dans l'étape de formation de seconde couche d'absorption, la seconde couche d'absorption (42) est formée sans fournir l'unité de courbage dans une région d'entrejambe (S3) qui est adaptée pour être en contact avec une entre jambe du porteur,
dans lequel l'unité de courbage est configurée par
une fente formée dans la première couche d'absorption (41), et
dans lequel dans l'étape de formation de première couche d'absorption,
une première unité de courbage incluant une première fente (45) et s'étendant dans la direction longitudinale est formée au centre de la région d'entrejambe (S3) dans la direction de la largeur, et
une deuxième unité de courbage incluant une paire de deuxièmes fentes (46) et s'étendant dans la direction longitudinale est formée à l'extérieur de la première unité de courbage dans la direction de la largeur, et
une troisième unité de courbage incluant une paire de troisièmes fentes (47) et s'étendant dans la direction longitudinale est formée à l'extérieur de la seconde unité de courbage dans la direction de la largeur.

2. Procédé selon la revendication 1, dans lequel dans l'étape de formation de seconde couche d'absorption, la seconde couche d'absorption (42) est disposée et formée de sorte qu'une région de l'unité de courbage formée dans la première couche d'absorption (41) soit couverte avec la seconde couche d'absorption (42), et
une autre région de l'unité de courbage est laissée ouverte sans être recouverte par la seconde couche d'absorption (42).

3. Procédé selon les revendications 1 ou 2, dans lequel dans l'étape de formation de seconde couche d'absorption, la seconde couche d'absorption est formée de sorte que la première unité de courbage formée dans la première couche d'absorption (41) soit couverte avec la seconde couche d'absorption (42), et
la seconde unité de courbage n'est pas couverte complètement par la seconde couche d'absorption (42), mais une partie de la seconde unité de courbage est laissée ouverte.

4. Procédé selon la revendication 1, dans lequel la première fente est formée au centre de la première couche d'absorption (41) dans la direction de la largeur, la paire de deuxièmes fentes est positionnée à l'extérieur de la première fente dans
la direction de la largeur, et la paire de troisièmes fentes est positionnée à l'extérieur de la paire de deuxièmes fentes dans la direction de la largeur.

5. Procédé selon l'une quelconque des revendications 1 à 4, comprenant en outre une étape de disposition d'élément élastique consistant à fournir un élément élastique (44) dans un état étiré dans la direction longitudinale de façon à recouvrir l'unité de courbage dans une direction d'épaisseur de l'absorbeur (40).

6. Procédé selon l'une quelconque des revendications 1 à 5, dans lequel au moins une de l'étape de formation de première couche d'absorption et l'étape de formation de seconde couche d'absorption inclut
une étape d'alimentation en matériau absorbant d'alimentation en un matériau absorbant configurant l'absorbeur (40), d'une matrice à déformation plastique qui inclut
une unité concave correspondant à une forme de la première couche d'absorption (41) ou la seconde couche d'absorption (42), et
un trou débouchant qui permet l'aspiration de l'air dans l'unité concave, et
une étape d'aspiration de formation de la première couche d'absorption (41) ou la seconde couche d'absorption (42) par aspiration du matériau absorbant.

7. Procédé selon l'une quelconque des revendications 1 à 6, dans lequel au moins une de l'étape de formation de première couche d'absorption et l'étape de formation de seconde couche d'absorption inclut
une étape d'alimentation en matériau absorbant d'alimentation en un matériau absorbant configurant l'absorbeur (40) d'une matrice à déformation plastique qui inclut
une unité concave correspond à une forme d'un agencement continu de la première couche d'absorption (41) ou la seconde couche d'absorption (42), et
un trou débouchant qui permet l'aspiration de l'air dans l'unité concave,
une étape d'aspiration de formation d'un corps continu de la première couche d'absorption (41) ou la seconde couche d'absorption (42) par aspiration du matériau absorbant, et
une étape de coupe de formation de la première couche d'absorption (41) ou la seconde couche d'absorption (42), par coupe du corps continu le long de la direction de la largeur.

8. Procédé selon la revendication 7, dans lequel
l'unité concave de la matrice à déformation plastique inclut une unité de base (216a) et une unité de paroi (216b) s'élevant de l'unité de base (216a), et le trou débouchant est formé dans l'unité de paroi (216b), et
dans l'étape d'aspiration, la première couche d'absorption (41) ou la seconde couche d'absorption (42) est formée par aspiration du matériau absorbant par l'intermédiaire au moins du trou débouchant dans l'unité de paroi (216b).

9. Procédé selon l'une quelconque des revendications 1 à 8, comprenant en outre :
une étape de formation de feuille supérieure de formation d'une feuille supérieure (10) ;
une étape de formation de feuille arrière de formation d'une feuille arrière ;
une étape de disposition de feuille auxiliaire de disposition d'une feuille auxiliaire (15) entre la feuille supérieure (10) et l'absorbeur (40) ; et
une étape de jonction de disposition de l'absorbeur (40) entre la feuille auxiliaire (15) et la feuille arrière, et de jonction ensuite de chacune de la feuille supérieure (10), la feuille auxiliaire (15), la feuille arrière, et l'absorbeur (40) dans une unité, dans lequel dans l'étape de disposition de feuille auxiliaire, la feuille auxiliaire (15) est agencée de sorte que les deux extrémités de la feuille auxiliaire dans la direction de la largeur soient positionnées près de l'unité de courbage.

10. Procédé selon l'une quelconque des revendications 1 à 9, comprenant en outre :
une étape d'alimentation en une feuille entre la première couche d'absorption (41) et la seconde couche d'absorption (42) avant que la première couche d'absorption (41) et la seconde couche d'absorption (42) ne soient disposées.

11. Procédé selon la revendication 6 à 9, comprenant en outre :
une étape d'alimentation en une feuille pour couvrir l'unité concave pour la formation de la première couche d'absorption ou la seconde couche d'absorption avant l'étape d'alimentation en matériau absorbant de sorte que le matériau absorbant constituant la seconde couche d'absorption (42) soit déposé sur la feuille.

12. Procédé selon la revendication 11, comprenant en outre :
une étape d'application d'adhésif thermofusible sur des surfaces opposées de la feuille pour lier les première et seconde couches d'absorption (41, 42) aux surfaces opposées de la feuille.

13. Procédé selon l'une quelconque des revendications 1 à 12, dans lequel la seconde couche d'absorption (42) est formée pour être exempte de fentes.
